(19) **Europäisches Patentamt / European Patent Office / Office européen des brevets**

(11) **EP 2 784 484 B1**

(12) **EUROPEAN PATENT SPECIFICATION**

(45) Date of publication and mention
of the grant of the patent:
**27.09.2017 Bulletin 2017/39**

(51) Int Cl.:
***G01N 21/27*** *(2006.01)*

(21) Application number: **14161517.9**

(22) Date of filing: **25.03.2014**

(54) **Optimized calibration method for determining the content of chlorophyll using pre-processing and independent component analysis**

Optimisierte Kalibrierungsmethode zur Bestimmung des Chlorophyll-Gehaltes mit Datenvorbehandlung und unabhängiger Komponentenanalyse

Procédé d'étalonnage optimisé pour déterminer le contenu en chlorophylle avec traitement préliminaire et analyse en composantes indépendantes

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2013 JP 2013065763**

(43) Date of publication of application:
**01.10.2014 Bulletin 2014/40**

(73) Proprietor: **Seiko Epson Corporation
Tokyo 163-0811 (JP)**

(72) Inventors:
• **Kurasawa, Hikaru
Nagano, 392-8502 (JP)**
• **Arai, Yoshifumi
Nagano, 392-8502 (JP)**

(74) Representative: **Hoffmann Eitle
Patent- und Rechtsanwälte PartmbB
Arabellastraße 30
81925 München (DE)**

(56) References cited:
• **DATABASE WPI Week 201316 Thomson Scientific, London, GB; AN 2013-C56663 XP002726655, & JP 2013 036973 A (SEIKO EPSON CORP) 21 February 2013 (2013-02-21)**

• **M. TOIVIAINEN ET AL: "Blind source separation in diffuse reflectance NIR spectroscopy using independent component analysis", JOURNAL OF CHEMOMETRICS, vol. 24, no. 7-8, 27 May 2010 (2010-05-27) , pages 514-522, XP055126170, ISSN: 0886-9383, DOI: 10.1002/cem.1316**
• **HARITOPOULOS M ET AL: "Image denoising using self-organizing map-based nonlinear independent component analysis", NEURAL NETWORKS, ELSEVIER SCIENCE PUBLISHERS, BARKING, GB, vol. 15, no. 8-9, 1 October 2002 (2002-10-01), pages 1085-1098, XP004385873, ISSN: 0893-6080, DOI: 10.1016/S0893-6080(02)00081-3**
• **R. M. JARVIS ET AL: "Genetic algorithm optimization for pre-processing and variable selection of spectroscopic data", BIOINFORMATICS, vol. 21, no. 7, 28 October 2004 (2004-10-28), pages 860-868, XP055126173, ISSN: 1367-4803, DOI: 10.1093/bioinformatics/bti102**
• **POLDER G ET AL: "Tomato sorting using independent component analysis on spectral images", REAL-TIME IMAGING, ACADEMIC PRESS LIMITED, GB, vol. 9, no. 4, 1 August 2003 (2003-08-01), pages 253-259, XP004473409, ISSN: 1077-2014, DOI: 10.1016/J.RTI.2003.09.008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- VASQUES G M ET AL: "Comparison of multivariate methods for inferential modeling of soil carbon using visible/near-infrared spectra", GEODERMA, ELSEVIER SCIENCE, AMSTERDAM, NL, vol. 146, no. 1-2, 31 July 2008 (2008-07-31), pages 14-25, XP023440037, ISSN: 0016-7061, DOI: 10.1016/J.GEODERMA.2008.04.007 [retrieved on 2008-06-10]

- GOMEZ A H ET AL: "Non-destructive measurement of acidity, soluble solids and firmness of Satsuma mandarin using Vis/NIR-spectroscopy techniques", JOURNAL OF FOOD ENGINEERING, BARKING, ESSEX, GB, vol. 77, no. 2, 1 November 2006 (2006-11-01), pages 313-319, XP027891085, ISSN: 0260-8774 [retrieved on 2006-11-01]

# EP 2 784 484 B1

**Description**

<u>BACKGROUND</u>

1. Technical Field

The present invention relates to a technique of creating a calibration curve, which is used to derive the content of a target component in a subject, from observation data of the subject, and a technique of calculating the content of the target component in the subject.

2. Related Art

A method has been proposed in which the concentration or the like of a target component is analyzed by performing independent component analysis of observation data, which is observed at a plurality of different positions of the subject, and expressing the observation data as a linear sum of a basic function with an independent component calculated by the independent component analysis as the basic function (refer to JP-A-2007-44104).

In the known technique described above, however, there is a problem in that a plurality of different pieces of observation data for a subject are required whenever a target component of the subject is measured and the measurement can not be accurately performed from a piece of observation data.

In addition, a variety of noises may be included in the observation data. In addition, depending on the subject, the observation data may be changed due to variations in the composition or the structure of the subject. In such a case, there is a problem in that the accuracy of independent component analysis or measurement using the same is reduced.

On the other hand, in order to prevent the reduction in accuracy of independent component analysis or accuracy of measurement using the same, there is a method of performing pre-processing to reduce noise or the variation in observation data. However, there are many methods for pre-processing. For this reason, there has been a problem in that it is difficult to know which pre-processing is suitable for the observation data and which pre-processing should be selected to perform accurate measurement.

TOIVIAINEN, M., Blind source separation in diffuse reflectance NIR spectroscopy using independent component analysis, J. Chemometrics 2010, vol. 24, pages 514-522, discloses the application of independent component analysis on near-infrared diffuse reflectance spectra and a three-phase preprocessing procedure.

ROGER, M. JARVIS, Genetic algorithm optimization for pre-processing and variable selection of spectroscopic data, Bioinformatics 2005, vol. 21, no. 7, pages 860-868, discloses a genetic algorithm for selecting the optimal combination of pre-processing methods when analysing spectroscopic data. It also mentions the possibility of combining many different pre-processing algorithms in order to find the most sensible combination.

<u>SUMMARY</u>

An advantage of some aspects of the invention is that accurate measurement from a piece of observation data regarding a subject can be achieved when measuring a target component of the subject.

The invention is directed to a method according to claim 1 and an apparatus according to claim 10.

Application Example 1

This application example is directed to a calibration curve creation method of creating a calibration curve, which is used to derive a content of a target component in a subject, from observation data of the subject. The calibration curve creation method includes: (a) acquiring the observation data for a plurality of samples of the subject; (b) acquiring the content of the target component in each sample; (c) executing pre-processing for the observation data of each sample, a pre-processing method is selected from a plurality of options; (d) estimating a plurality of independent components when separating the pre-processed observation data of each sample into a plurality of independent components and calculating a mixing coefficient corresponding to the target component for each sample based on the plurality of independent components; and (e) calculating a regression equation of the calibration curve based on the content of the target component of each of the plurality of samples and the mixing coefficient of each sample . In the process (c), the pre-processing includes first pre-processing including processing for correcting the observation data and second pre-processing including whitening, and a plurality of processing methods are prepared as processing methods of each of the first pre-processing and the second pre-processing and the pre-processing method is set by combining one or more of the processing methods of each of the first pre-processing and the second pre-processing. The process (d) includes: (i) calculating an independent component matrix including the independent component of each sample; (ii) calculating an estimated mixing matrix, which indicates a set of vectors defining a ratio of an independent component element of each independent component in each sample, from the independent component matrix; and (iii) calculating a correlation between each of the vectors included in the estimated mixing matrix and the content of the target component of each of the plurality of samples and select the vector, which is determined to have the highest correlation, as a mixing coefficient corresponding to the target component. In the process (i), the first pre-processing, the second pre-processing, and independent component analysis processing are executed in this order using the pre-processing method selected in the process (c).

According to the calibration curve creation method of Application Example 1, for a plurality of samples of the subject, the calibration curve for deriving the amount of target component included in the subject from the observation data of the subject is created from the content of the target component and the observation data acquired from each sample. For this reason, if this calibration curve is used, the content of the target component can be accurately calculated even if the number of pieces of observation data of the subject is one. Therefore, if the calibration curve is created in advance according to the calibration curve creation method of Application Example 1, it is sufficient to acquire a piece of observation data for the subject at the time of measurement. As a result, the amount of target component can be accurately calculated from a piece of observation data that is an actual measurement value. In addition, since an estimated mixing matrix is calculated and a vector highly correlated with the content of the target component of the sample is extracted from the estimated mixing matrix, it is possible to obtain the mixing coefficient with high estimation accuracy.

In addition, since the appropriate pre-processing is selected and executed according to the characteristics of the observation data of the subject, information included in the observation data of the subject can be appropriately extracted. As a result, it is possible to improve the measurement accuracy.

Application Example 2

This application example is directed to the calibration curve creation method according to Application Example 1, wherein in the process (c), the processing methods of the first pre-processing includes a projection on null space.

According to this configuration, it is possible to improve the measurement accuracy by reducing the baseline variation of the observation data by pre-processing based on the projection on null space.

Application Example 3

This application example is directed to the calibration curve creation method according to Application Example 1, wherein in the process (c), the processing methods of the first pre-processing includes centering.

According to this configuration, since it is possible to align the baseline of the observation data by subtracting the average value of the observation data by pre-processing based on the centering, it is possible to improve the measurement accuracy.

Application Example 4

This application example is directed to the calibration curve creation method according to Application Example 1, wherein in the process (c), the processing methods of the first pre-processing includes normalization.

According to this configuration, since it is possible to reduce the variation in the observation data due to changes in the measurement conditions by setting the average value of the observation data to 0 and the variance to 1 by pre-processing based on the normalization, it is possible to improve the measurement accuracy.

Application Example 5

This application example is directed to the calibration curve creation method according to Application Example 1, wherein in the process (c), the processing methods of the first pre-processing includes smoothing processing.

According to this configuration, since it is possible to reduce unnecessary random noise included in the observation data by pre-processing based on the smoothing processing, it is possible to improve the measurement accuracy.

Application Example 6

This application example is directed to the calibration curve creation method according to Application Example 1, wherein in the process (c), the processing methods of the first pre-processing includes differential spectrum processing.

According to this configuration, since it is possible to emphasize the variation component of the observation data by pre-processing based on the differential spectrum processing, it is possible to improve the measurement accuracy.

Application Example 7

This application example is directed to the calibration curve creation method according to Application Example 1, wherein in the process (c), the processing methods of the first pre-processing includes differential processing.

According to this configuration, since it is possible to extract a variation portion of the observation data by pre-processing based on the differential processing, it is possible to improve the measurement accuracy.

Application Example 8

This application example is directed to the calibration curve creation method according to Application Example 1, wherein in the process (c), the processing methods of the second pre-processing includes a principal component analysis.

According to this configuration, since it is possible to perform orthogonalization and dimensional reduction of the observation data by pre-processing based on the principal component analysis, the independent component analysis processing of the process (d) can be accurately performed at high speed.

Application Example 9

This application example is directed to the calibration curve creation method according to Application Example 1, wherein in the process (c), the processing methods of the second pre-processing includes a factor analysis.

According to this configuration, since it is possible to perform orthogonalization and dimensional reduction considering the random noise included in the observation data by pre-processing based on the factor analysis, the independent component analysis processing of the process (d) can be accurately performed at high speed.

Application Example 10

This application example is directed to a calibration curve creation apparatus that creates a calibration curve, which is used to derive a content of a target component in a subject, from observation data of the subject. The calibration curve creation apparatus includes: a sample observation data acquisition unit that acquires the observation data for a plurality of samples of the subject; a sample target component amount acquisition unit that acquires the content of the target component in each sample; a pre-processing method selection unit that selects a processing method of a pre-processing of the observation data from a plurality of options, the pre-processing includes first pre-processing including correction processing and second pre-processing including whitening; a mixing coefficient estimation unit that estimates a plurality of independent components when separating the observation data of each sample into a plurality of independent components and calculates a mixing coefficient corresponding to the target component for each sample based on the plurality of independent components; and a regression equation calculation unit that calculates a regression equation of the calibration curve based on the content of the target component of each of the plurality of samples and the mixing coefficient of each sample. A plurality of processing methods are prepared as processing methods of each of the first pre-processing and the second pre-processing, and the pre-processing method selection unit combines one or more of the processing methods of each of the first pre-processing and the second pre-processing to set the pre-processing method having a plurality of options and selects an optimal combination from the set pre-processing method. The mixing coefficient estimation unit includes: an independent component matrix calculation section that calculates an independent component matrix including the independent component of each sample; an estimated mixing matrix calculation section that calculates an estimated mixing matrix, which indicates a set of vectors defining a ratio of an independent component element of each independent component in each sample, from the independent component matrix; and a mixing coefficient selection section that calculates a correlation between each of the vectors included in the estimated mixing matrix and the content of the target component of each of the plurality of samples and selects the vector, which is determined to have the highest correlation, as a mixing coefficient corresponding to the target component. The independent component matrix calculation section calculates the independent component matrix by executing the first pre-processing, the second pre-processing, and independent component analysis processing in this order using the pre-processing method selected by the pre-processing method selection unit.

According to the calibration curve creation apparatus of Application Example 10, similar to the calibration curve creation method of Application Example 1, it is sufficient to acquire a piece of observation data for the subject at the time of measurement. Therefore, an effect that the amount of target component can be accurately calculated from a piece of observation data, which is an actual measurement value, is obtained. In addition, since the appropriate pre-processing is selected and executed according to the characteristics of the observation data by the pre-processing method selection unit, information included in the observation data can be appropriately extracted by the mixing coefficient estimation unit. As a result, it is possible to improve the measurement accuracy.

Application Example 11

This application example is directed to the calibration curve creation apparatus according to Application Example 10, wherein the pre-processing method selection unit includes a projection on null space as an option of the processing method of the first pre-processing.

According to this configuration, it is possible to improve the measurement accuracy by reducing the baseline variation of the observation data by pre-processing based on the projection on null space.

Application Example 12

This application example is directed to the calibration curve creation apparatus according to Application Example 10, wherein the pre-processing method selection unit includes centering as an option of the processing method of the first pre-processing.

According to this configuration, since it is possible to align the baseline of the observation data by subtracting the average value of the observation data by pre-processing based on centering, it is possible to improve the measurement accuracy.

Application Example 13

This application example is directed to the calibration curve creation apparatus according to Application Example 10, wherein the pre-processing method selection unit includes normalization as an option of the processing method of the first pre-processing.

According to this configuration, since it is possible to reduce the variation in the observation data due to changes in the measurement conditions by setting the average value of the observation data to 0 and the variance to 1 by pre-processing based on normalization, it is possible to improve the measurement accuracy.

Application Example 14

This application example is directed to the calibration curve creation apparatus according to Application Example 10, wherein the pre-processing method selection unit includes smoothing processing as an option of the processing method of the first pre-processing.

According to this configuration, since it is possible to reduce unnecessary random noise included in the observation data by pre-processing based on the smoothing processing, it is possible to improve the measurement accuracy.

Application Example 15

This application example is directed to the calibration curve creation apparatus according to Application Example 10, wherein the pre-processing method selection unit includes differential spectrum processing as an option of the processing method of the first pre-processing.

According to this configuration, since it is possible to emphasize the variation component of the observation data by pre-processing based on the differential spectrum processing, it is possible to improve the measurement accuracy.

Application Example 16

This application example is directed to the calibration curve creation apparatus according to Application Example 10, wherein the pre-processing method selection unit includes differential processing as an option of the processing method of the first pre-processing.

According to this configuration, since it is possible to extract a variation portion of the observation data by pre-processing based on the differential processing, it is possible to improve the measurement accuracy.

Application Example 17

This application example is directed to the calibration curve creation apparatus according to Application Example 10, wherein the pre-processing method selection unit includes a principal component analysis as an option of the processing method of the second pre-processing.

According to this configuration, since it is possible to perform orthogonalization and dimensional reduction of the observation data by pre-processing based on the principal component analysis, the calculation of the independent component matrix calculation section can be accurately performed at high speed.

Application Example 18

This application example is directed to the calibration curve creation apparatus according to Application Example 10, wherein the pre-processing method selection unit includes a factor analysis as an option of the processing method of the second pre-processing.

According to this configuration, since it is possible to perform orthogonalization and dimensional reduction considering the random noise included in the observation data by pre-processing based on the factor analysis, the calculation of the independent component matrix calculation section can be accurately performed at high speed.

Application Example 19

This application example is directed to the calibration curve creation apparatus according to Application Example 10, wherein the calibration curve creation apparatus further includes a storage unit that stores the independent component matrix calculated by the independent component matrix calculation section, a target component rank indicating at which position of the estimated mixing matrix the mixing coefficient selected by the mixing coefficient selection section is present, and a regression equation calculated by the regression equation calculation unit.

According to this configuration, the calibration curve creation apparatus can store the independent component matrix, the target component rank, and the regression equation in the storage unit.

Application Example 20

This application example is directed to a target component gauging apparatus that calculates a content of a target component in a subject. The target component gauging apparatus includes: a subject observation data acquisition unit that acquires observation data of the subject; a data-for-measurement acquisition unit that acquires measurement data including at least an independent component corresponding to the target component; a mixing coefficient calculation unit that calculates a mixing coefficient with respect to the target component for the subject based on the measurement data and the observation data of the subject; and a target component amount calculation unit that calculates the content of the target component based on a constant of a regression equation indicating a relationship between a content and a mixing coefficient corresponding to the target component, which is prepared in advance, and the mixing coefficient calculated by the mixing coefficient calculation unit. The mixing coefficient calculation unit executes a pre-processing method, which is selected by a pre-processing method selection unit of a calibration curve creation apparatus that calculates the independent component, as first pre-processing including processing for correcting the observation data and second pre-processing including whitening, in this order.

According to the target component gauging apparatus, the content of the target component in the subject can be accurately calculated just by acquiring a piece of observation data regarding the subject.

Application Example 21

This application example is directed to the target component gauging apparatus according to Application Example 20, wherein the data-for-measurement acquisition unit acquires an independent component, which corresponds to the target component and is calculated in advance, as the measurement data, and the mixing coefficient calculation unit calculates an inner product of the independent component and the observation data of the subject and sets an value of the inner product as the mixing coefficient.

According to the target component gauging apparatus, a mixing coefficient highly correlated with the target component of the subject can be accurately and easily calculated.

Application Example 22

This application example is directed to the target component gauging apparatus according to Application Example 20, wherein the data-for-measurement acquisition unit acquires, as the data for measurement, a plurality of independent components when separating observation data of a plurality of samples into a plurality of independent components, and the mixing coefficient estimation unit calculates an estimated mixing matrix for the subject based on the observation data of the subject and the plurality of independent components, and extracts a mixing coefficient corresponding to the target component from the calculated estimated mixing matrix.

According to the target component gauging apparatus, a mixing coefficient highly correlated with the target component of the subject can be accurately calculated.

In addition, the invention can be realized in various forms other than those described above. For example, the invention can also be realized in a form as a target component gauging apparatus that stores the regression line calculated by the calibration curve creation method in a memory, a form as a computer program to realize as a function the configuration of each unit included in the target component gauging apparatus, and a storage medium (non-transitory storage medium) on which the computer program or the computer program is recorded.

BRIEF DESCRIPTION OF THE DRAWINGS

The invention will be described with reference to the accompanying drawings, wherein like numbers reference like elements.

Fig. 1 is a flowchart showing a calibration curve creation method as one embodiment.
Fig. 2 is a graph showing the relationship between the wavelength of light and the spectral reflectance for green vegetables having different freshness.
Fig. 3A is an explanatory diagram showing a personal computer and its peripheral devices that are used in steps 4 and 5.
Fig. 3B is a functional block diagram of an apparatus used in steps 4 and 5.
Fig. 3C is a functional block diagram showing an example of the internal configuration of an independent component matrix calculation section.
Fig. 4 is an explanatory diagram showing an example of the combination of pre-processing that can be selected.
Fig. 5 is an explanatory diagram schematically showing a measured data set stored in a hard disk drive.
Fig. 6 is a flowchart showing the mixing coefficient estimation process executed by a CPU.
Fig. 7 is an explanatory diagram for explaining an estimated mixing matrix.
Fig. 8 is an explanatory diagram showing an example of a scatter plot with high correlation.
Fig. 9 is an explanatory diagram showing an example of a graph of a scatter plot with low correlation.
Fig. 10 is a flowchart showing the regression equation calculation process executed by the CPU of the computer.
Fig. 11 is a functional block diagram of an apparatus used when measuring a target component.
Fig. 12 is a flowchart showing the target component measuring process executed by the CPU of the computer.
Fig. 13 is an explanatory diagram showing the measurement accuracy due to differences in pre-processing.

DESCRIPTION OF EXEMPLARY EMBODIMENTS

Hereinafter, embodiments of the invention will be described in the following order.

A. Calibration curve creation method
B. Target component measuring method
C. Various algorithms and influences on the measurement accuracy
D. Modification examples

In the present embodiment, the following abbreviations are used.

- ICA: independent component analysis
- SNV: standard normal variate transformation
- PNS: projection on null space
- PCA: principal component analysis
- FA: factor analysis

Hereinafter, embodiments of the invention will be described. An embodiment is related to a method of creating the calibration curve for deriving the chlorophyll content in green vegetables from the spectrum of the spectral reflectance of the green vegetables as observation data. The green vegetables are spinach, Chinese cabbage, and a green pepper, for example.

A. Calibration curve creation method

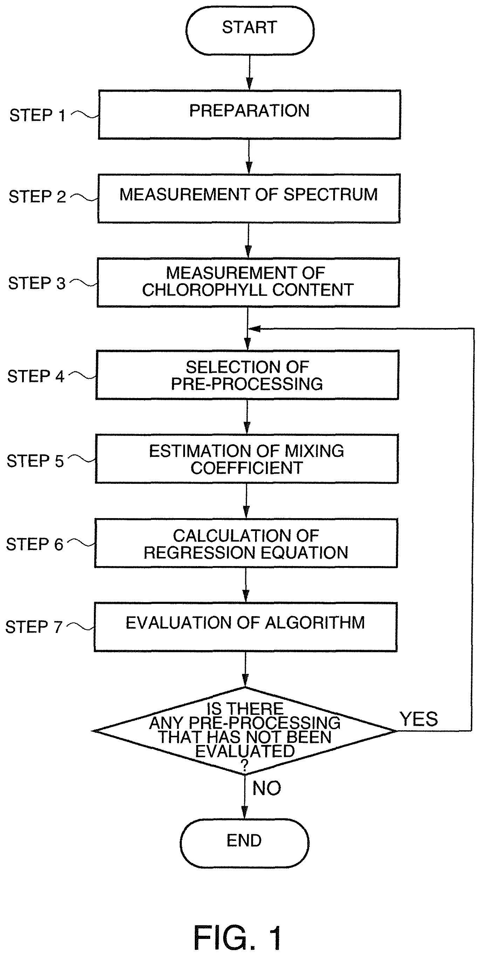

Fig. 1 is a flowchart showing a calibration curve creation method as an embodiment. As shown in Fig. 1, this calibration curve creation method includes seven steps of steps 1 to 7. The steps 1 to 7 are performed in this order. The steps 1 to 7 will be described in order.

Step 1

The step 1 is a preparatory step, and is performed by the operator. The operator prepares a plurality of green vegetables (for example, spinach) of the same type, which have different freshness, as samples. In the present embodiment, n (n is an integer of 2 or more) samples are used.

Step 2

The step 2 is a spectrum measurement step, and is performed by the operator using a spectrometer. The operator measures the spectrum of the spectral reflectance for each sample by imaging each of the plurality of samples prepared in step 1 using the spectrometer. The spectrometer is a known instrument that measures a spectrum by making light from a measured object be transmitted through a spectroscope and receiving the spectrum output from the spectroscope on the imaging surface of an imaging device. The relationship expressed as in the following Expression (1) is satisfied between the spectrum of the spectral reflectance and the spectrum of absorbance.

$$[\text{Absorbance}] = -\log_{10}[\text{Reflectance}] \quad \cdots \quad (1)$$

The spectrum of the measured spectral reflectance is converted into the absorbance spectrum using Expression (1) . Conversion into the absorbance spectrum is performed because a linear combination needs to be established in the mixed signal analyzed in the independent component analysis, which will be described later, and the linear combination is established for the absorbance from the Lambert-Beer's law. Therefore, in step 2, it is also possible to measure the absorbance spectrum instead of the spectral reflectance spectrum. As a measurement result, data of absorbance distribution showing the characteristics with respect to the wavelength of the measured object is output. The data of absorbance distribution is also referred to as spectral data.

Specifically, in step 2, the operator images a predetermined portion for each sample, and measures the spectrum of the predetermined portion. The predetermined portion may be any portion in each sample, but a portion having freshness that is not greatly different from that of the entire sample is preferable. For example, when the freshness of a certain portion in a sample is extremely low, a portion excluding the portion with low freshness is set as a predetermined portion to be measured.

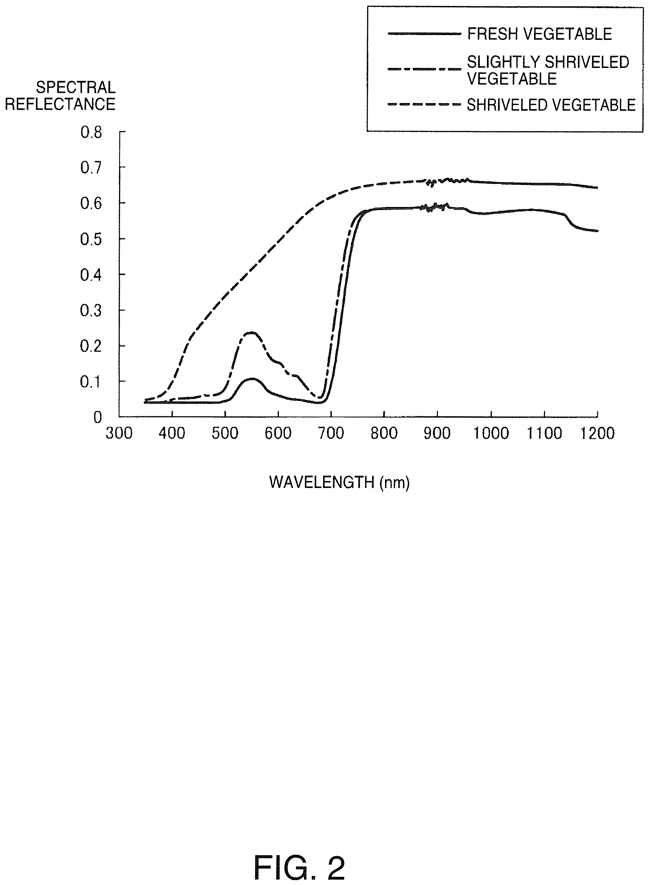

Fig. 2 is a graph showing the relationship between the wavelength of light and the spectral reflectance for green vegetables having different freshness. As shown in Fig. 2, the spectrum waveforms of fresh vegetable, slightly shriveled vegetable, and shriveled vegetable are different. In the case of the fresh vegetable or the slightly shriveled vegetable, the reflectance decreases abruptly in a wavelength range equal to or less than about 700 nm. This is because light absorption by chlorophyll occurs at a wavelength of 700 nm or less. On the other hand, in the case of the shriveled vegetable, the reflectance rises greatly in a wavelength range of 700 nm or less because chlorophyll has decreased. Thus, since a spectrum waveform changes with the freshness of green vegetables, the spectrum for each sample is measured in step 2.

In addition, instead of measuring the spectral reflectance spectrum or the absorbance spectrum using a spectroscope, it is possible to estimate these spectra from other measured values. For example, it is also possible to measure a sample with a multi-band camera and estimate the spectral reflectance or the absorbance spectrum from the obtained multi-band image. As such an estimation method, for example, a method disclosed in JP-A-2001-99710 can be used.

Step 3

The step 3 is a step of measuring the chlorophyll content, and is performed by the operator. The operator measures the chlorophyll content, which is the content of a target component in each sample, by chemically analyzing each of the plurality of samples prepared in step 1. Specifically, a predetermined portion is extracted from each sample, chlorophyll that is a target component is extracted from the predetermined portion, and the chlorophyll content is measured. Although the "predetermined portion" may be any portion of the sample, it is preferable that the "predetermined portion" be the same as the portion in which the spectrum has been measured in step 2.

Step 4

The step 4 is a pre-processing selection step, and is performed using a personal computer.

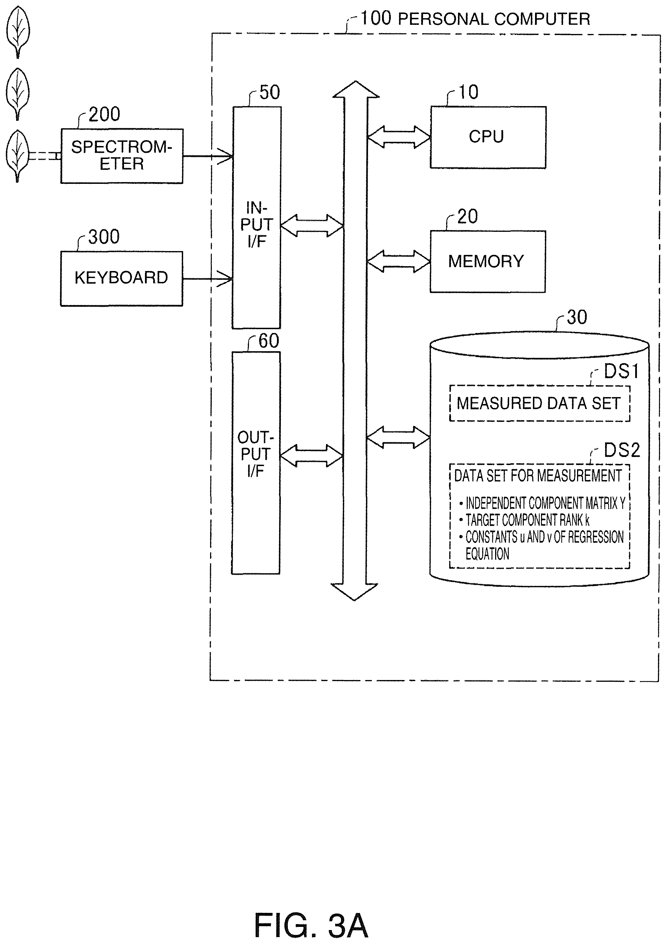

Fig. 3A is an explanatory diagram showing a personal computer 100 and its peripheral devices that are used in step 4 and steps 5 to 7, which will be described later. As shown in Fig. 3A, the personal computer (hereinafter, simply referred to as a "computer") 100 is electrically connected to a spectrometer 200 and a keyboard 300.

The computer 100 is a known apparatus including a CPU 10 that executes various kinds of processes and control when executing a computer program (hereinafter, simply referred to as a "program"), a memory 20 (storage unit) that is a data storage location, a hard disk drive 30 that stores a program or data and information, an input interface (I/F) 50, and an output interface (I/F) 60.

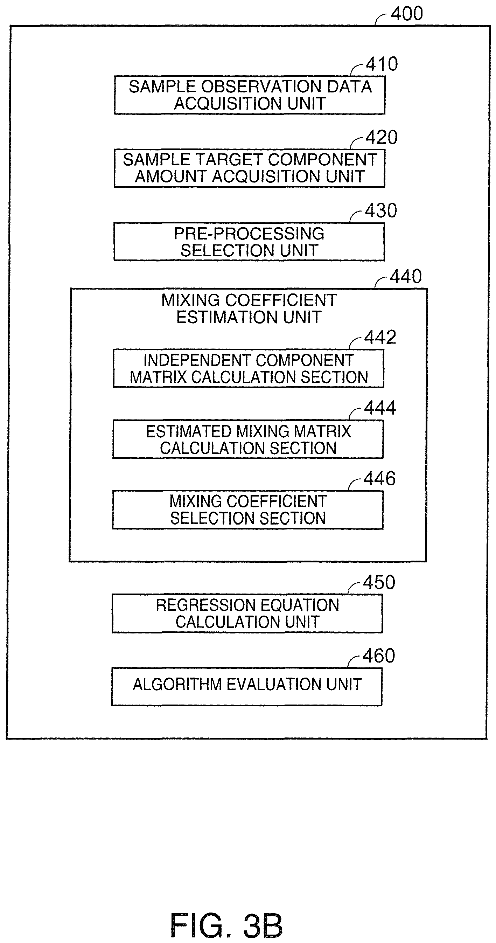

Fig. 3B is a functional block diagram of an apparatus used in steps 4 to 6. This apparatus 400 includes a sample observation data acquisition unit 410, a sample target component amount acquisition unit 420, a pre-processing selection unit 430, a mixing coefficient estimation unit 440, a regression equation calculation unit 450, and an algorithm evaluation unit 460. The mixing coefficient estimation unit 440 includes an independent component matrix calculation section 442, an estimated mixing matrix calculation section 444, and a mixing coefficient selection section 446. In addition, the sample observation data acquisition unit 410 and the sample target component amount acquisition unit 420 are realized by the cooperation of the CPU 10 and the input I/F 50 and the memory 20 shown in Fig. 3A, for example. The pre-processing selection unit 430, the mixing coefficient estimation unit 440, the independent component matrix calculation section 442, the estimated mixing matrix calculation section 444, and the mixing coefficient selection section 446 are realized by the cooperation of the CPU 10 and the memory 20 shown in Fig. 3A, for example. In addition, the regression equation calculation unit 450 and the algorithm evaluation unit 460 are realized by the cooperation of the CPU 10 and the memory 20 shown in Fig. 3A, for example. In addition, each of these units or sections can be realized by other specific devices or hardware circuits excluding the personal computer shown in Fig. 3A.

The step 4 is a step of selecting the combination of pre-processing, and is performed by a personal computer.

A first pre-processing section 470 can select processing from variations of standard normal variate transformation (SNV) 472 and projection on null space (PNS) 474 and perform the pre-processing in combination.

The SNV 472 is a process for obtaining normalized data, in which the average value is 0 and the standard deviation is 1, by subtracting the average value of data to be processed and dividing the result by the standard deviation.

The PNS 474 is a process for removing a baseline variation included in the data to be processed. In the measurement of the spectrum, a variation between data called a baseline variation, such as an increase or decrease in the average value of data, occurs in the measurement data due to various factors. For this reason, it is preferable to remove the variation factors before performing the independent component analysis. The PNS can be used as pre-processing that can remove any baseline variation.

Assuming that the order of the target baseline variation is zero-order, first-order, and second-order, the PNS can remove the baseline variation of any combination thereof.

In addition, the PNS is described in Zeng-Ping Chen, Julian Morris, and Elaine Martin, "Extracting Chemical Information from Spectral Data with Multiplicative Light Scattering Effects by Optical Path-Length Estimation and Correction", 2006, for example.

Although the PNS is a method for removing the variation of the baseline whose influence changes algebraically functionally in the data length direction, it depends on target measurement data which-order algebraic function influence is to be removed. Therefore, there is a plurality of variations in the PNS depending on a method of selecting the baseline to be removed.

In addition, when performing the SNV 472 on the spectral data obtained in step 2 of Fig. 1, there is no need to perform the process by the PNS 474. On the other hand, when performing the process by the PNS 474, it is preferable to perform certain normalization processing (for example, the SNV 472) thereafter.

In addition, as the first pre-processing, it is possible to perform processing other than the SNV or the PNS. In the first pre-processing, it is preferable to perform certain normalization processing, but the normalization processing may be omitted. The first pre-processing section 470 is also referred to as a "correction processing section" hereinbelow. Details of these two processes 472 and 474 will be further described later.

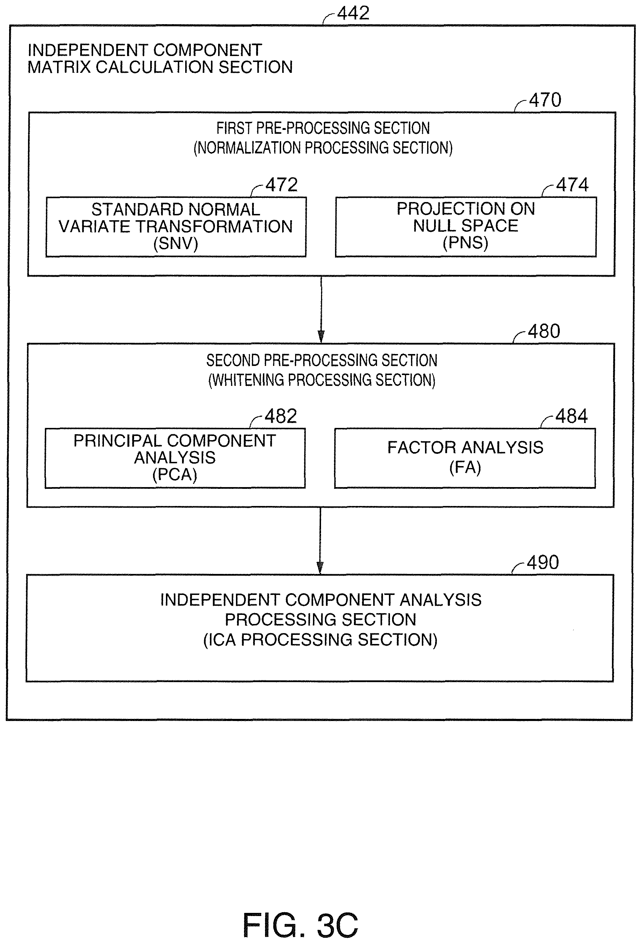

A second pre-processing section 480 can perform pre-processing using either a principal component analysis (PCA) 482 or a factor analysis (FA) 484. In addition, as the second pre-processing, it is possible to use processing other than the PCA or the FA. The second pre-processing section 480 is also referred to as a "whitening processing section" hereinbelow. In a general ICA method, dimensional compression of data to be processed and decorrelation are performed as the second pre-processing. Since a transformation matrix to be calculated by the ICA is limited to an orthogonal transformation matrix by the second pre-processing, it is possible to reduce the amount of calculation in the ICA. Such second pre-processing is called "whitening", and the PCA is used in many cases. In the PCA, however, when random noise is included in the data to be processed, an erroneous result may be obtained due to the influence. Therefore, in order to reduce the influence of random noise, it is preferable to perform the whitening using the FA, which has robustness against noise, instead of the PCA. The second pre-processing section 480 shown in Fig. 3C can select either the PCA or the FA to perform the whitening. Details of these two processes 482 and 484 will be further described later. In addition, the whitening processing may be omitted.

As the pre-processing, it is preferable to select an appropriate combination of processes from the above processes according to the characteristics of observation data and perform the selected combination of processes. In order to determine which combination of processes is appropriate, possible combinations of pre-processing are evaluated, and the most accurate combination is selected as the pre-processing. In order to find a combination of pre-processing that is optimal for the target sample observation data, the regression equation of the calibration curve is calculated for each combination, and the accuracy is evaluated.

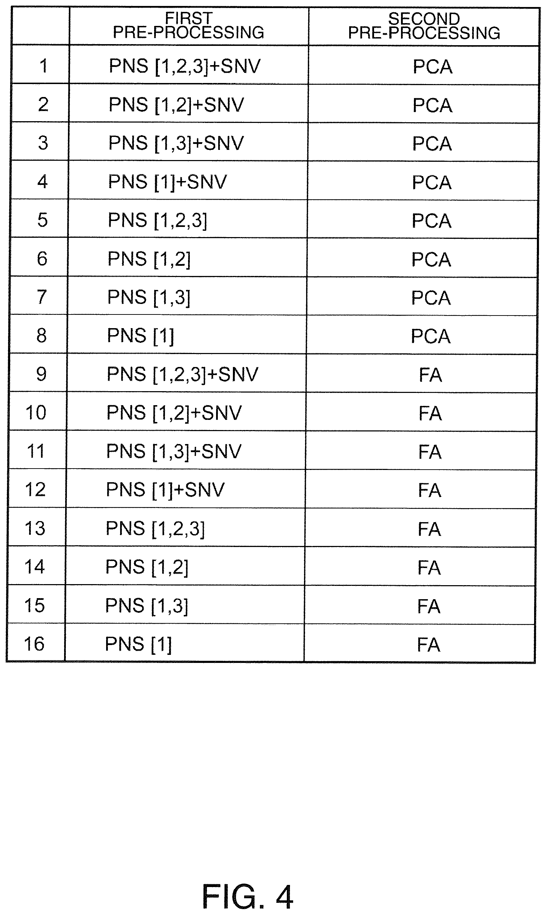

When the SNV and the PNS are used in the first pre-processing and the PCA and the FA are used in the second pre-processing, examples of the combination of pre-processing shown in Fig. 4 can be considered.

In step 4, these combinations of pre-processing are selected sequentially from the start, and are executed for the observation data. For the pre-processed observation data, the regression equation of the calibration curve is obtained through steps 5 and 6 to be described later. Then, the accuracy is evaluated in step 7. These steps are repeated to evaluate the accuracy for all combinations of pre-processing and select an optimal combination of pre-processing.

Although the result of pre-processing is evaluated to select pre-processing in the present embodiment, other methods may be used as a method of selecting pre-processing. The operator may select pre-processing from the list of pre-processing.

Step 5

The step 5 is a step of estimating a mixing coefficient, and is performed using a personal computer.

Fig. 3C is a functional block diagram showing an example of the internal configuration of the independent component matrix calculation section 442. The independent component matrix calculation section 442 includes the first pre-processing section 470, the second pre-processing section 480, and an independent component analysis processing section 490. A plurality of pre-processing methods are prepared for the first pre-processing section 470 and the second pre-processing section 480. In actual processing, some of the plurality of pre-processing methods are selected and are performed in combination. The three processing sections 470, 480, and 490 calculate an independent component matrix (to be described later) by processing the data to be processed (absorbance spectrum in the present embodiment) in this order. Details of the processing of the respective sections will be described later.

The spectrometer 200 shown in Fig. 3A is used in step 2. The computer 100 acquires the absorbance spectrum obtained from the spectral distribution measured by the spectrometer 200 in step 2, as spectral data, through the input I/F 50 (corresponding to the sample observation data acquisition unit 410 shown in Fig. 3B). In addition, the computer 100 acquires the chlorophyll content measured in step 3 through the input I/F 50 in response to the operation of the keyboard 300 by the operator (corresponding to the sample target component amount acquisition unit 420 shown in Fig. 3B). In addition, the chlorophyll content measured in step 3 may be input to the computer 100 as a mass of chlorophyll per unit mass (for example, per 100 g) of a predetermined portion in which chlorophyll has been measured. Alternatively, the chlorophyll content may be input as an absolute value of the mass.

As a result of the acquisition of the spectral data and the chlorophyll content described above, a data set including the spectral data and the chlorophyll content (hereinafter, referred to as a "measured data set") DS1 is stored in the hard disk drive 30 of the computer 100.

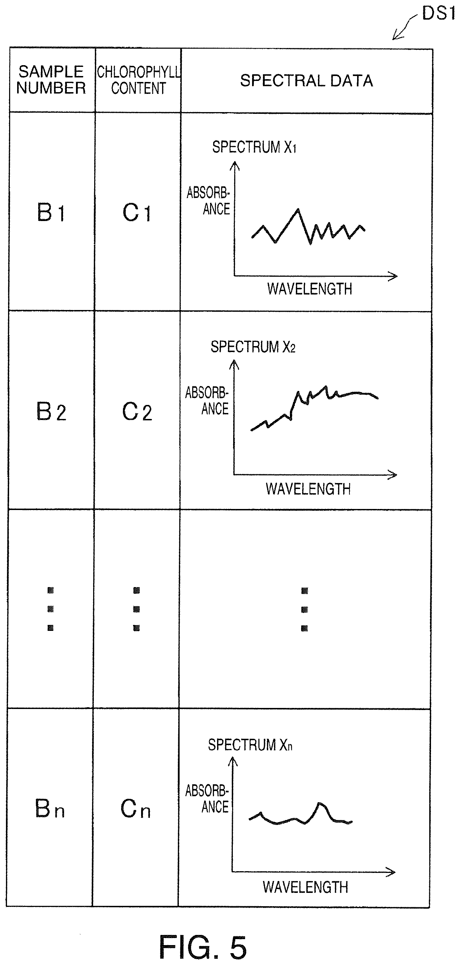

Fig. 5 is an explanatory diagram schematically showing the measured data set DS1 stored in the hard disk drive 30. As shown in Fig. 5, the measured data set DS1 is a data structure including sample numbers B1, B2, ..., Bn for identifying a plurality of samples prepared in step 1, chlorophyll content C1, C2, ..., Cn of each sample, and spectral data $X_1$, $X_2$, ..., $X_n$ of each sample. In the measured data set DS1, the chlorophyll content C1, C2, ..., Cn and the spectral data $X_1$, $X_2$, ..., $X_n$ are matched with the sample numbers B1, B2, ..., Bn so that the corresponding sample thereof can be seen.

The CPU 10 loads a predetermined program stored in the hard disk drive 30 to the memory 20 and executes the program to perform a process for estimating the mixing coefficient that is the operation of step 4. The predetermined program can also be downloaded from the outside using a network, such as the Internet. In step 4, the CPU 10 functions as the mixing coefficient estimation unit 440 shown in Fig. 3B.

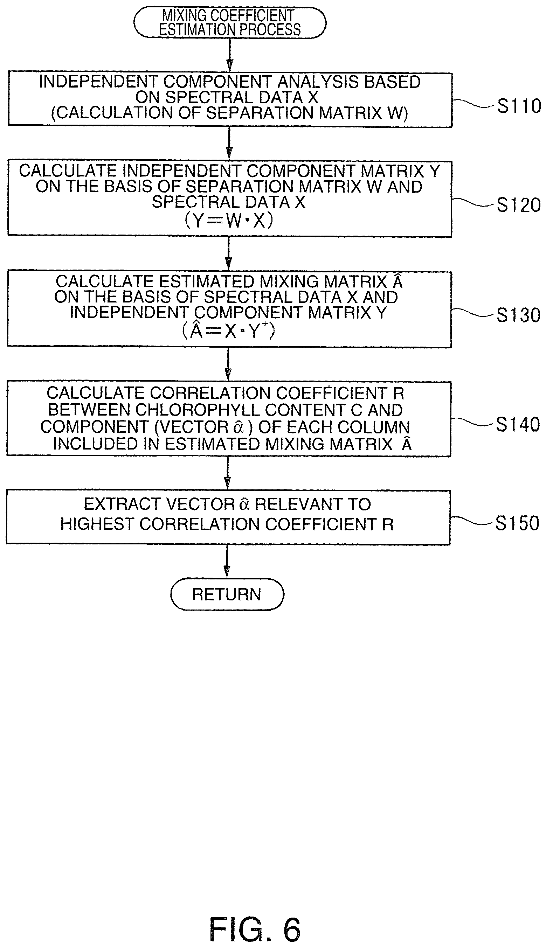

Fig. 6 is a flowchart showing the mixing coefficient estimation process executed by the CPU 10. When the process starts, the CPU 10 performs independent component analysis first (step S110).

The independent component analysis (ICA) is one of the multi-dimensional signal analysis methods, and is a technique for observing a mixed signal, in which independent signals overlap each other, in some different conditions and separating the original independent signals based on the result. By using the independent component analysis, the spectrum as an independent component can be estimated from the spectral data (observation data) obtained in step 2 by regarding the spectral data obtained in step 2 as mixed data of "m" (unknown) independent components including the spectrum due to chlorophyll.

In the present embodiment, the independent component analysis is performed when the three processing sections 470, 480, and 490 shown in Fig. 3C perform their processes in this order.

Subsequent to the first pre-processing of the first pre-processing section 470 and the second pre-processing of the second pre-processing section 480, the independent component analysis processing of the independent component analysis processing section (ICA processing section) 490 is performed.

Although a plurality of methods are prepared for the first pre-processing and the second pre-processing, it depends on target measurement data which pre-processing is appropriate. Therefore, each process in each combination of pre-processing prepared is performed for evaluation, and pre-processing determined to have the highest performance is used eventually.

The independent component analysis processing section (ICA processing section) 490 estimates the spectrum as an independent component by performing the ICA on the spectral data subjected to the first pre-processing and the second pre-processing. Generally, in the ICA, a high-order statistic indicating the independence of separated pieces of data is used as an indicator for the separation of independent components (independence indicator). For example, kurtosis is a typical independence indicator. In addition to kurtosis, it is also possible to use indicators, such as $\beta$ divergence, as independence indicators of ICA.

Next, the typical processing of independent component analysis will be described in detail. It is assumed that the spectrum S (hereinafter, this spectrum may be simply referred to as an "unknown component") of "m" unknown components (source) is given as a vector of the following Expression (2) and "n" spectral data X obtained in step 2 is given as a vector of the following Expression (3). Each element $(S_1, S_2, ..., S_m)$ included in Expression (2) is a vector (spectrum). That is, for example, an element $S_1$ is expressed as in Expression (4). Elements $(X_1, X_2, ..., Xn)$ included in Expression (3) are also vectors. For example, the element $X_1$ is expressed as in Expression (5). Subscript 1 is the number of wavelength ranges where the spectrum has been measured. In addition, the number of elements m of the spectrum S of unknown components is an integer of 1 or more, and is determined experimentally or empirically in advance according to the type (here, spinach) of sample.

$$S = [S_1, S_2, ..., S_m]^T \cdots (2)$$

$$X = [X_1, X_2, ..., X_n]^T \cdots (3)$$

$$S_1 = \{S_{11}, S_{12}, ..., S_{11}\} \cdots (4)$$

$$X_1 = \{X_{11}, X_{12}, ..., X_{11}\} \cdots (5)$$

Each unknown component is assumed to be statistically independent. The relationship of the following Expression (6) is satisfied between the unknown component S and the spectra data X.

$$X = A \cdot S \cdots (6)$$

A in Expression (6) is a mixing matrix, and can be expressed as in the following Expression (7). Although the letter "A" needs to be expressed in bold as shown in Expression (7), the letter "A" is expressed in a normal letter herein from the limitation of letters used in the specification. Hereinafter, other bold letters representing the matrix are also similarly expressed in normal letters.

$$A = \begin{pmatrix} a_{11} & \cdots & a_{1m} \\ \vdots & \ddots & \vdots \\ a_{n1} & \cdots & a_{nm} \end{pmatrix} \cdots (7)$$

The mixing coefficient $a_{ij}$ included in the mixing matrix A indicates the degree of contribution of the unknown component $S_j$ (j = 1 to m) to the spectral data $X_i$ (i = 1 to n) that is observation data.

When the mixing matrix A is known, the least square solution of the unknown component S can be easily calculated as $A^+ \cdot X$ by using a pseudo- inverse matrix $A^+$ of A. In the present embodiment, however, since the mixing matrix A is unknown, the unknown component S and the mixing matrix A should be estimated only from the observation data X. That is, as shown in the following Expression (8), a matrix showing the spectrum as an independent component (hereinafter, referred to as an "independent component matrix") Y is calculated only from the observation data X using the separation matrix W (m × n). As an algorithm for calculating the separation matrix W in the following Expression (8), it is possible to adopt various algorithms, such as Infomax, Fast Independent Component Analysis (FastICA), and Joint Approximate Diagonalization of Eigenmatrices (JADE).

$$Y = W \cdot X \quad \cdots \quad (8)$$

The independent component matrix Y corresponds to the estimate of the unknown component S. Therefore, the following Expression (9) can be obtained, and the following Expression (10) can be obtained by transforming Expression (9) .

$$X = \hat{A} \cdot Y \quad \cdots \quad (9)$$

$$\hat{A} = X \cdot Y^{+} \quad \cdots \quad (10)$$

The estimated mixing matrix ΛA obtained by Expression (10) (written in this manner from the limitation of letters used in the specification, but means a signed letter on the left side of Expression (10) in practice. The same for the other letters) can be expressed as in the following Expression (11).

$$\hat{A} = \begin{pmatrix} \hat{a}_{11} & \cdots & \hat{a}_{1m} \\ \vdots & \ddots & \vdots \\ \hat{a}_{n1} & \cdots & \hat{a}_{nm} \end{pmatrix} \quad \cdots \quad (11)$$

In step S110 of Fig. 6, the CPU 10 performs up to the process for calculating the separation matrix W described above. Specifically, the separation matrix W is calculated using one of the algorithms, such as Infomax, FastICA, and JADE described above, based on the input of the spectral data X of each sample obtained in step 2 and stored in advance in the hard disk drive 30. In addition, as shown in Fig. 3C described above, it is preferable to perform the normalization processing of the first pre-processing section 470 and the whitening processing of the second pre-processing section 480 as pre-processing of independent component analysis.

After the execution of step S110, the CPU 10 performs processing for calculating the independent component matrix Y based on the separation matrix W and the spectral data X of each sample, which is obtained in step 2 and is stored in advance in the hard disk drive 30 (step S120). In this calculation processing, calculation is performed according to Expression (8) described above. In the processing of steps S110 and 5120, the CPU 10 functions as the independent component matrix calculation section 442 shown in Fig. 3B.

Then, the CPU 10 performs processing for calculating the estimated mixing matrix ΛA based on the spectral data X of each sample stored in advance in the hard disk drive 30 and the independent component matrix Y calculated in step 5120 (step S130). In this calculation processing, calculation is performed according to Expression (10) described above.

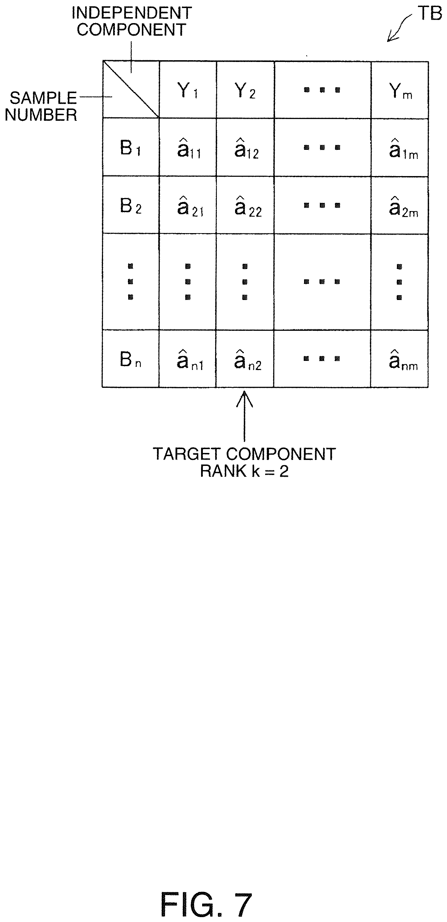

Fig. 7 is an explanatory diagram for explaining the estimated mixing matrix ΛA. As shown in Fig. 7, table TB has sample numbers $B_1$, $B_2$, ..., $B_n$ in a vertical direction and elements of the independent component matrix Y (hereinafter, referred to as "independent component elements") $Y_1$, $Y_2$, ..., $Y_m$ in a horizontal direction. The element in the table TB determined by the sample number $B_i$ (i = 1 to n) and the independent component element $Y_j$ (j = 1 to m) is the same as the coefficient $\Lambda a_{ij}$ (refer to Expression (11)) included in the estimated mixing matrix ΛA. Also from the table TB, it can be seen that the coefficient $\Lambda a_{ij}$ included in the estimated mixing matrix ΛA indicates the ratio of the independent component elements $Y_1$, $Y_2$, ... , $Y_m$ in each sample. A target component rank k illustrated in Fig. 7 will be described later. In the processing of step S130, the CPU 10 functions as the estimated mixing matrix calculation section 444 shown in Fig. 3B.

The estimated mixing matrix ΛA is obtained by the processing up to step S130. That is, the coefficient (estimated mixing coefficient) $\Lambda a_{ij}$ included in the estimated mixing matrix ΛA is obtained. Then, the process proceeds to step S140.

In step S140, CPU 10 calculates a correlation (degree of similarity) between the chlorophyll content C1, C2, ..., Cn measured in step 3 and components (hereinafter, referred to as a vector Λα) of each column included in the estimated mixing matrix ΛA calculated in step S130. Specifically, a correlation between the chlorophyll content C (C1, C2, ..., Cn) and the vector $\Lambda\alpha_1$ ($\Lambda a_{11}$, $\Lambda a_{21}$, ..., $\Lambda a_{n1}$) of the first column is calculated, and then a correlation between the chlorophyll content C (C1, C2, ..., Cn) and the vector $\Lambda\alpha_2$ ($\Lambda a_{12}$, $\Lambda a_{22}$, ..., $\Lambda a_{n2}$) of the second column is calculated. In this manner, a correlation between the chlorophyll content C and the vector of each column is sequentially calculated, and a correlation between the chlorophyll content C (C1, C2, ..., Cn) and the vector $\Lambda\alpha_m$ ($\Lambda a_{1m}$, $\Lambda a_{2m}$, ..., $\Lambda a_{nm}$) of the m-th column is finally calculated.

Such a correlation can be calculated by using a correlation coefficient R according to the following Expression (12). The correlation coefficient R is called a Pearson's product-moment correlation coefficient.

$$R = \frac{\sum_{i=1}^{n}(C_i - \overline{C})(\hat{a}_{ik} - \overline{\hat{\alpha}_k})}{\sqrt{\sum_{i=1}^{n}(C_i - \overline{C})^2}\sqrt{\sum_{i=1}^{n}(\hat{a}_{ik} - \overline{\hat{\alpha}_k})^2}} \quad \cdots \quad (12)$$

where $\overline{C}$ and $\overline{\hat{\alpha}_k}$ are the chlorophyll content and the average value of the vector $\hat{\alpha}_k$, respectively

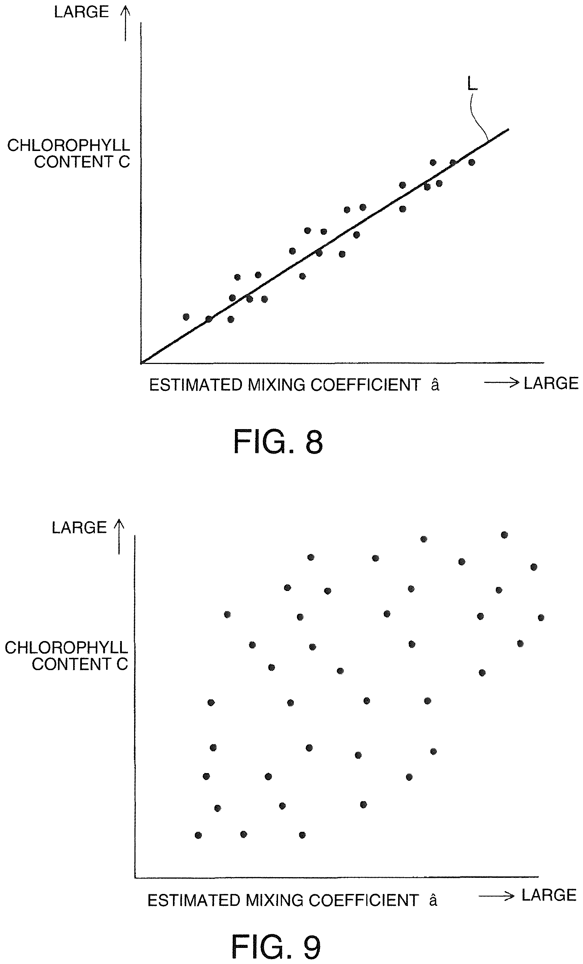

Fig. 8 is a graph of the scatter plot. In the scatter plot shown in Fig. 8, the vertical axis indicates the chlorophyll content C, and the horizontal axis indicates the coefficient (hereinafter, referred to as an "estimated mixing coefficient") $\Lambda a$ of the estimated mixing matrix $\Lambda A$. The scatter plot shown in Fig. 8 is obtained by plotted points determined from the elements C1, C2, ..., Cn of the chlorophyll content C and estimated mixing coefficients $\Lambda a_{1j}$, $\Lambda a_{2j}$, ..., $\Lambda a_{nj}$ (j = 1 to m) included in the vector $\Lambda\alpha$ of the estimated mixing matrix $\Lambda A$ in the vertical direction. In the example shown in Fig. 8, plotted points are gathered relatively near the straight line L. In this case, the correlation between the chlorophyll content C and the estimated mixing coefficient $\Lambda a$ is high. In contrast, if the correlation between the chlorophyll content C and the estimated mixing coefficient $\Lambda a$ is low, as shown in Fig. 9, plotted points are not located linearly but spread. That is, the higher the correlation between the chlorophyll content C and the estimated mixing coefficient $\Lambda a$, the higher the tendency in which plotted points are gathered linearly. The correlation coefficient R shown in Expression (12) indicates the degree of tendency in which plotted points are gathered linearly.

As a result of step S140 of Fig. 6, a correlation coefficient $R_j$ (j = 1, 2, ..., m) for each independent component (independent component spectrum) $Y_j$ is obtained. Then, the CPU 10 specifies a correlation coefficient with the highest correlation, that is, a correlation coefficient with a value close to 1, from the correlation coefficient $R_j$ obtained in step S140. In the scatter plot described above, the correlation coefficient $R_j$ at which plotted points are gathered most linearly is specified. Then, a column vector $\Lambda\alpha$ when the highest correlation coefficient R is obtained is selected from the estimated mixing matrix $\Lambda A$ (step S150).

The selection in step S150 means selecting a column from a plurality of columns in the table TB shown in Fig. 7. Elements of the selected column are mixing coefficients of the independent component corresponding to chlorophyll that is a target component. As a result of the selection, a vector $\Lambda\alpha_k$ ($\Lambda a_{1k}$, $\Lambda a_{2k}$, ..., $\Lambda a_{nk}$) is obtained. Here, k is assumed to be an integer of 1 to m. In addition, the value of k is temporarily stored in the memory 20 as a target component rank indicating which number of independent component corresponds to the target component. $\Lambda a_{1k}$, $\Lambda a_{2k}$, ..., $\Lambda a_{nk}$ included in the vector $\Lambda\alpha_k$ correspond to the "mixing coefficient corresponding to the target component" in Application Example 1. In addition, in the example shown in Fig. 7, the target component rank k = 2 indicates a column vector $\Lambda\alpha_2$ = ($\Lambda a_{12}$, $\Lambda a_{22}$, ..., $\Lambda a_{n2}$) corresponding to the independent component $Y_2$. In this specification, the term "rank" is used to mean a "value indicating the position within the matrix". In processing of step S140 and S150, the CPU 10 functions as the mixing coefficient selection section 446 shown in Fig. 3B. After the execution of step S150, the CPU ends the process of calculating the mixing coefficient. As a result, step 5 is completed, and the process proceeds to step 6.

Step 6

The step 6 is a step of calculating the regression equation, and is performed using the computer 100 in the same manner as when performing step 5. In step 6, the computer 100 performs processing for calculating the regression equation of the calibration curve. In addition, data up to step 5 may be transferred to another computer to perform step 6.

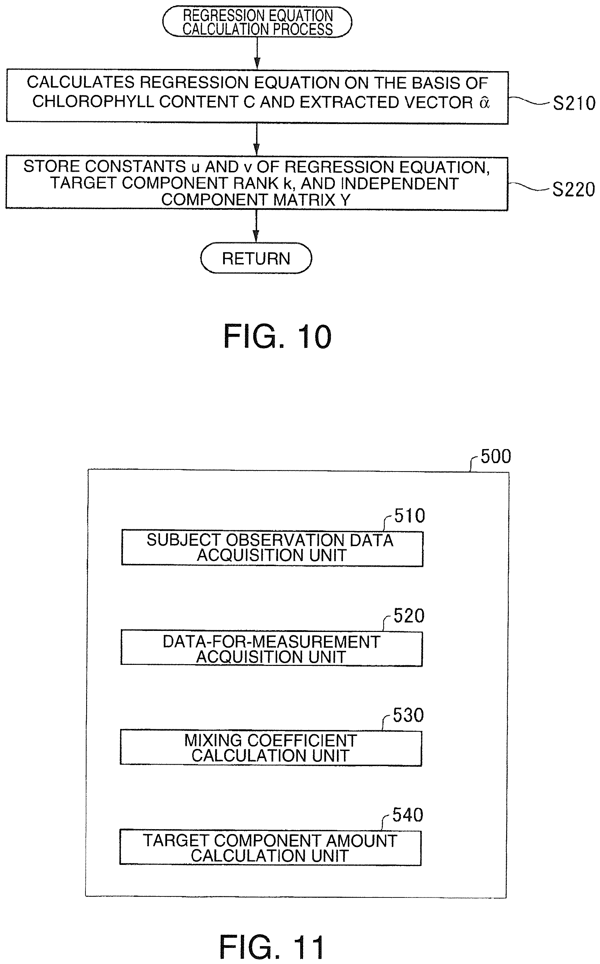

Fig. 10 is a flowchart showing the regression equation calculation process executed by the CPU 10 of the computer 100. When the processing starts, CPU 10 calculates a regression equation F first based on the chlorophyll content C (C1, C2, ..., Cn) measured in step 3 and the vector $\Lambda\alpha_k$ ($\Lambda a_{1k}$, $\Lambda a_{2k}$, ..., $\Lambda a_{nk}$) selected in step S150 (step S210). When the scatter plot shown in Fig. 8 has a highest correlation, the straight line L in Fig. 8 corresponds to the regression equation F. Since a method of calculating the regression equation is known, detailed explanation thereof will not be given. For example, the straight line L is calculated using the least square method so that the distance (residual) from the straight line L to each plotted point becomes close to 0. The regression equation F can be expressed as in the following Expression (13). In step S210, constants u and v in Expression (13) are calculated.

$$F : C = u\hat{\alpha}_k + v \quad \cdots \quad (13)$$

After the execution of step S210, the CPU 10 stores a combination method of the constants u and v of the regression equation F calculated in step S210, the target component rank k (Fig. 7) obtained in step S150, the independent component matrix Y calculated in step S120 of the mixing coefficient calculation process (Fig. 6), and the pre-processing selected in the pre-processing selection in the hard disk drive 30 as a data set for measurement DS2 (step S220). Then, the CPU 10 proceeds to "return" to temporarily end the process of calculating the regression equation. As a result, it is possible to obtain the regression line of the calibration curve, and the calibration curve creation method shown in Fig. 1 also ends. In the processing of steps S210 and S220, the CPU 10 functions as the regression equation calculation unit 450 shown in Fig. 3B.

Step 7

The step 7 is an algorithm evaluation step, and is performed using the computer 100 in the same manner as when performing steps 5 and 6.

One of the combinations of pre-processing is selected in step 4, mixing coefficient calculation processing is performed, and the regression line of the calibration curve is calculated. The accuracy of the calibration curve in this case is evaluated, it is evaluated how much the combination of pre-processing selected in step 4 is effective for the observation data, and the combination of pre-processing selected in step 4 is compared with other combinations of pre-processing. A correlation coefficient between the mixing coefficient and the true value can be used for the evaluation. A result when calculating the measurement accuracy SEP by measuring the sample data using the calibration curve can be used.

Based on the evaluation result, a combination of pre-processing with the highest accuracy among the combinations of pre-processing evaluated up to now is determined as a candidate of pre-processing. When there is a combination of pre-processing that has not been evaluated yet, the process returns to step 4 to evaluate the next pre-processing. When the evaluation of all pre-processing ends, the current pre-processing candidate is adopted as pre-processing for the target observation data.

B. Target component measuring method

Next, the target component measuring method will be described. A subject is assumed to contain the same components as a sample used when creating the calibration curve. Specifically, the target component measuring method is performed using a computer. In addition, the computer herein may be the computer 100 used when creating the calibration curve, or may be a different computer.

Fig. 11 is a functional block diagram of an apparatus used when measuring a target component. An apparatus 500 includes a subject observation data acquisition unit 510, a data-for-measurement acquisition unit 520, a mixing coefficient calculation unit 530, and a target component amount calculation unit 540. The mixing coefficient calculation unit 530 includes a pre-processing section 532. This pre-processing section 532 has functions of both the first pre-processing section 470 and second pre-processing section 480 shown in Fig. 3C, and performs pre-processing selected in the calibration curve creation. The subject observation data acquisition unit 510 is realized by the cooperation of the CPU 10 and the input I/F 50 and the memory 20 shown in Fig. 3A, for example. The data-for-measurement acquisition unit 520 is realized by the cooperation of the CPU 10 and the memory 20 and the hard disk drive 30 shown in Fig. 3A, for example. The mixing coefficient calculation unit 530 and the target component amount calculation unit 540 are realized by the cooperation of the CPU 10 and the memory 20 shown in Fig. 3A, for example. The computer to realize each function shown in Fig. 11 is assumed to be the computer 100 used when creating the calibration curve, and the data set for measurement DS2 described above is stored in a storage unit, such as a hard disk drive.

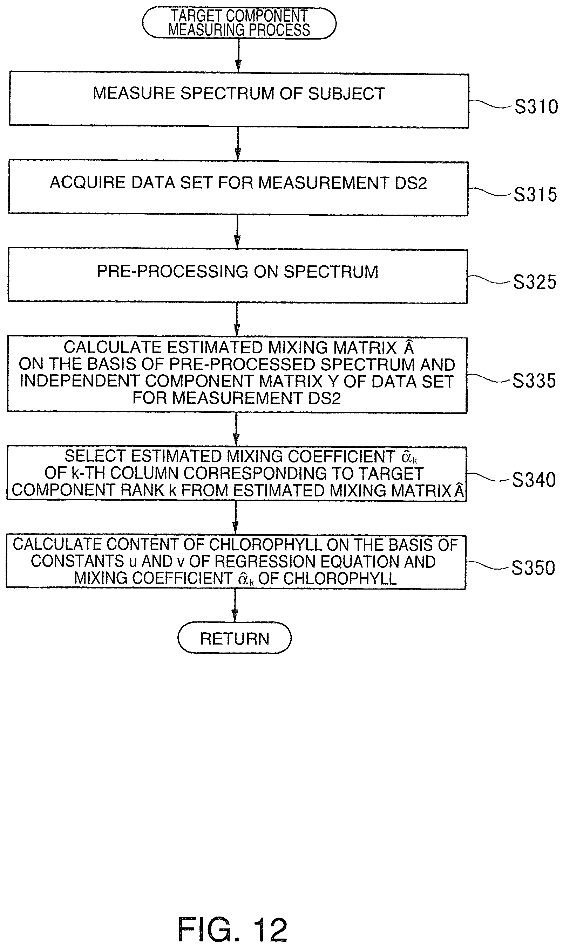

Fig. 12 is a flowchart showing the target component measuring process executed by the CPU 10 of the computer 100. The target component measuring process is realized when the CPU 10 loads a predetermined program stored in the hard disk drive 30 to the memory 20 and executes the program. As shown in Fig. 12, when the process starts, the CPU 10 first performs processing for imaging a green vegetable, which is a subject, using a spectrometer (step S310). The imaging in step S310 can be performed as in step 2. As a result, the absorbance spectrum Xp of the subject is obtained. The spectrometer used in the measurement process is preferably the same model as the spectrometer that is used in the creation of the calibration curve in order to suppress error. In order to further suppress the error, it is more preferable that the spectrometer used in the measurement process be the same apparatus as the spectrometer used in the creation of the calibration curve. In addition, as in step 2 of Fig. 1, instead of measuring the spectral reflectance spectrum or the absorbance spectrum using a spectroscope, it is possible to estimate these spectra from other measured values. The spectrum Xp of the absorbance of the subject obtained when imaging a subject once is expressed as a vector as in the following Expression (14).

$$X_p = \{X_{p1}, \ X_{p2}, \ ..., \ X_{pl}\} \quad \cdots \quad (14)$$

In the processing of step S310, the CPU 10 functions as the subject observation data acquisition unit 510 shown in Fig. 11. Then, the CPU 10 acquires the data set for measurement DS2 from the hard disk drive 30, and stores the data set for measurement DS2 in the memory 20 (step S315).

In the processing of step S315, the CPU 10 functions as the data-for-measurement acquisition unit 520 shown in Fig. 11.

After the execution of step S315, pre-processing is performed on the absorbance spectrum Xp of the subject obtained in step S310 (step 5325). As this pre-processing, the same processing as the pre-processing (that is, the normalization processing of the first pre-processing section 470 and the whitening processing of the second pre-processing section 480) used in step 4 of Fig. 1 (more specifically, step S110 of Fig. 6) when creating the calibration curve is performed based on the combination of pre-processing included in the data set for measurement.

Then, the CPU 10 performs processing for calculating the estimated mixing matrix AA regarding the subject based on the independent component matrix Y included in the data set for measurement DS2 and the pre-processed spectrum obtained instep S325 (step S335). Specifically, arithmetic processing according to Expression (10) described above is performed. The estimated mixing matrix AA is obtained by calculating the inverse matrix (pseudo-inverse matrix) $Y^+$ of the independent component matrix Y included in the data set for measurement DS2 and multiplying the pre-processed spectrum obtained in step S325 by the pseudo-inverse matrix $Y^+$.

As shown in the following Expression (15), the estimated mixing matrix AA in the measurement process is a row vector ("1 × m" matrix) configured to include mixing coefficients corresponding to the respective independent components. After the execution of step S335, the CPU 10 reads the target component rank k included in the data set for measurement DS2 from the hard disk drive 30, extracts the mixing coefficient $\Lambda\alpha_k$ of the k-th component corresponding to the target component rank k from the estimated mixing matrix AA calculated in step S335, and temporarily stores the mixing coefficient $\Lambda\alpha_k$ in the memory 20 as a mixing coefficient of chlorophyll that is a target component (step S340). In the processing of steps S325, S335, and S340, the CPU 10 functions as the mixing coefficient calculation unit 530 shown in Fig. 11.

$$\hat{A} = (\hat{\alpha}_1, \hat{\alpha}_2, \cdots, \hat{\alpha}_m) \quad \cdots \quad (15)$$

Then, the CPU 10 reads the constants u and v of the regression equation included in the data set for measurement DS2 from the hard disk drive 30, and calculates the content C of chlorophyll by substituting the constants u and v and the mixing coefficient $\Lambda\alpha_k$ of chlorophyll as a target component, which is obtained in step S340, into the right side of Expression (13) (step S350). The content C is calculated as a mass of chlorophyll per unit mass (for example, per 100 g) of the subject. In the processing of step S350, the CPU 10 functions as the target component amount calculation unit 540 shown in Fig. 11. Then, the process proceeds to "return" to end the target component measuring process.

In the present embodiment, the content C (mass per unit mass) calculated in step S350 is used as the content of chlorophyll in the subject. However, instead of this, the content C calculated in step S350 may be corrected using the normalization coefficient used in the normalization of step S325 and the corrected value may be used as the content to be calculated. Specifically, the absolute value (gram) of the content may be calculated by multiplying the content C by the standard deviation. According to this configuration, it is possible to calculate the content C more accurately depending on the type of target component.

According to the calibration curve creation method of the embodiment configured as described above, the chlorophyll content can be accurately calculated from one spectrum that is an actual measurement value of the green vegetable as a subject.

C. Various algorithms and influences on the measurement accuracy

Hereinafter, various algorithms used in the first pre-processing section 470, the second pre-processing section 480, and the independent component analysis processing section 490 shown in Fig. 3C and the influences on the measurement accuracy will be described in order.

The difference in accuracy by the combination of pre-processing is shown using actual observation data as an example. Food data is used as a target.

C-1. First pre-processing (normalization processing using SNV/PNS)

As the first pre-processing performed by the first pre-processing section 470, standard normal variate transformation (SNV) and projection on null space (PNS) can be used.

The SNV is given as the following Expression (16).

$$z = \frac{x - x_{ave}}{\sigma} \quad \cdots \quad (16)$$

Here, z is data after processing, x is data to be processed (absorbance spectrum in the present embodiment), $x_{ave}$ is the average value of the data to be processed x, and $\sigma$ is a standard deviation of the data to be processed x. As a result of standard normal variate transformation, the normalized data z whose average value is 0 and standard deviation is 1 is obtained.

By performing the PNS, it is possible to reduce the baseline variation included in the data to be processed. In the measurement of data to be processed (absorbance spectrum in the present embodiment), a variation between data called a baseline variation, such as an increase or decrease in the average value of data, occurs in the measurement data due to various factors. For this reason, it is preferable to remove the variation factors before performing the independent component analysis (ICA). The PNS can be used as pre-processing that can reduce any baseline variation of the data to be processed. In particular, for the measurement data of the absorbed light spectrum or the reflected light spectrum including an infrared region, the advantage of applying the PNS is large since such a baseline variation occurs frequently. The principle of removing the baseline variation, which is included in data obtained by measurement (simply referred to as "measurement data x"), by the PNS will be described below. In addition, as a typical example, a case will be described in which the measurement data is an absorbed light spectrum or a reflected light spectrum including an infrared region will be described. However, the PNS can also be similarly applied for other types of measurement data (for example, sound data) .

Generally, in an ideal system, the measurement data x (data to be processed x) is expressed as in the following Expression (17) using "m" (m is an integer of 2 or more) independent components $s_i$ (i = 1 to m) and each mixture ratio $C_i$.

$$x = \sum_{i=1}^{m} c_i s_i \quad \cdots \quad (17)$$
$$= A \cdot s$$

Here, A is a matrix (mixing matrix) formed with the mixture ratio $c_i$ .

Also in the independent component analysis (ICA), processing is performed on the assumption that this model is used. However, there are various variation factors (condition of a sample, changes in the measurement environment, and the like) in actual measurement data. Therefore, as a model that takes these variation factors into consideration, a model that expresses the measurement data x as in the following Expression (18) can be considered.

$$x = b \sum_{i=1}^{m} c_i s_i + aE + d\lambda + e\lambda^2 + \varepsilon \quad \cdots \quad (18)$$

Here, b is a parameter indicating a variation in the amplitude direction of the spectrum, a, d, and e are parameters indicating the amount of constant baseline variation E (also referred to as "average value variation"), the amount of variation $\lambda$ that linearly depends on the wavelength, and the amount of variation $\lambda^2$ that depends on the square of the wavelength, respectively, and $\varepsilon$ is other variation components. In addition, the constant baseline variation E is given as E = {1, 1, 1, ..., 1}T, and is a constant vector whose data length is equal to the data length (the number of segments of the wavelength range) of the measurement data x. The variations $\lambda$ and $\lambda^2$ depending on the wavelength are given as $\lambda = \{\lambda_1, \lambda_2, ...,\lambda_N\}$T and $\lambda^2 = \{\lambda_{12}, \lambda_{22}, ..., \lambda_{N2}\}$T, respectively. Here, N is the data length of the measurement data x. In addition, as a variation depending on the wavelength, third-order or higher variations can also be taken into consideration. In general, it is possible to take into consideration up to the g-th order variation $\lambda^g$ (g is an integer of 2 or more).

In the PNS, data in which the baseline variation components E, $\lambda$, $\lambda^2$, ..., $\lambda^g$ (g is an integer of 2 or more) have been reduced can be obtained by considering the space including the baseline variation components E, $\lambda$, $\lambda^2$, ... , $\lambda^g$ and proj ecting the measurement distance x to the space (null space) that does not include these variation components. As specific calculation, the data z after processing of the PNS is calculated by the following Expression (19).

$$z = (1 - PP^+)x = b\sum_{i=1}^{m} c_i k_i + \varepsilon^*$$

$$P = \{1, \lambda, \lambda^2 \cdots \lambda^g\} \qquad \cdots \quad (19)$$

Here, $P^+$ is a pseudo-inverse matrix of P. $k_i$ is a result obtained by projecting the component $s_i$ of Expression (18) to the null space not including that does not include variation components. In addition, $\varepsilon^*$ is a result obtained by projecting the variation component $\varepsilon$ of Expression (18) to the null space.

In addition, by performing normalization (for example, the SNV) after processing of the PNS, it is also possible to eliminate the influence of the variation b in the amplitude direction of the spectrum in Expression (18).

An independent component obtained by performing the ICA on the data pre-processed by the PNS is an estimate of the component $k_i$ of Expression (19), which is different from the true component $s_i$. However, since the mixture ratio $c_i$ is not changed from the value in original Expression (18), there is no influence on the measurement process (Fig. 12) that uses the mixture ratio $c_i$. Thus, since the true component $s_i$ cannot be obtained by the ICA if the PNS is performed as pre-processing of the ICA, the idea of applying the PNS as pre-processing of the ICA is not possible normally. In the present embodiment, however, there is no influence on the measurement process even if the PNS is performed as pre-processing of the ICA. If the PNS is performed as the pre-processing, it is possible to perform measurement more accurately.

The order of the variation to be removed by the PNS can be removed in any combination. Since these variations are error factors in the ICA or the measurement, removing the variations in advance is desirable in many cases. However, not only the variation components but also information required for the measurement may be removed together. Depending on the characteristics of the observation data, it may be better to leave the required information even if there are variations in order to improve the measurement accuracy. Therefore, as the processing of the PNS, when zero-order, first-order, and second-order variations are considered, it is possible to remove variation component combinations, such as [zero-order, first-order, second-order], [zero-order, first-order], [zero-order, second-order], and [zero-order], for example.

In addition, details of the PNS is described in Zeng-Ping Chen, Julian Morris, and Elaine Martin, "Extracting Chemical Information from Spectral Data with Multiplicative Light Scattering Effects by Optical Path-Length Estimation and Correction", 2006, for example.

C-2. Second pre-processing (whitening processing using PCA/FA)

As second pre-processing performed by the second pre-processing section 480, principal component analysis (PCA) and factor analysis (FA) can be used.

In a general ICA method, dimensional compression of data to be processed and decorrelation are performed as pre-processing. Since a transformation matrix to be calculated by the ICA is limited to an orthogonal transformation matrix by this pre-processing, it is possible to reduce the amount of calculation in the ICA. Such pre-processing is called "whitening", and the PCA is used in many cases. The whitening using the PCA is described in detail in Chapter 6 of Aapo Hyvarinen, Juha Karhumen, Erkki Oja, "Independent Comonent Analysis", 2001, John Wiley & Sons, Inc., for example.

In the PCA, however, when random noise is included in the data to be processed, an erroneous result may be obtained due to the influence of the random noise. Then, in order to reduce the influence of random noise, it is preferable to perform the whitening using the factor analysis (FA), which has robustness against noise, instead of the PCA. Hereinafter, the principle of the whitening using the FA will be described.

As described above, generally, in the ICA, a linear mixture model (above Expression (17)) that expresses the data to be processed x as a linear sum of the component $s_i$ is assumed, and the mixture ratio $c_i$ and the component $s_i$ are calculated. However, random noise as well as the component $s_i$ is added to actual data in many cases. Therefore, as a model that takes random noise into consideration, a model that expresses the measurement data x as in the following Expression (20) can be considered.

$$X = A \cdot s + \rho \quad \cdots \quad (20)$$

Here, $\rho$ is random noise.

In addition, it is possible to obtain an estimation of the mixing matrix A and the independent component $s_i$ by performing whitening considering the noise mixture model and then performing the ICA.

In the FA of the present embodiment, it is assumed that the independent component $s_i$ and the random noise $\rho$ follow the normal distribution N(0, Im) and N(0, E), respectively. In addition, as is generally known, the first parameter x1 of the normal distribution N(x1, x2) indicates an expected value, and the second parameter x2 indicates a standard deviation. In this case, since the data to be processed x is a linear sum of the variable according to the normal distribution, the data to be processed x also follows the normal distribution. Here, assuming that the covariance matrix of the data to be processed x is V[x], the normal distribution that the data to be processed x follow can be expressed as N(0, V[x]). In this case, the likelihood function regarding the covariance matrix V[x] of the data to be processed x can be calculated by the following procedure.

First, assuming that the independent components $s_i$ are perpendicular to each other, the covariance matrix V[x] of the data to be processed x is calculated by the following Expression (21).

$$\mathrm{V}[\mathrm{x}] = \mathrm{E}[\mathrm{xx}^T] = \mathrm{AA}^T + \Sigma \quad \cdots \quad (21)$$

Here, $\Sigma$ is a covariance matrix of the noise $\rho$.

Thus, the covariance matrix V[x] can be expressed by the mixing matrix A and the covariance matrix $\Sigma$ of noise. In this case, the logarithmic likelihood function L(A, $\Sigma$) is given as the following Expression.

$$L(A,\Sigma) = -\frac{n}{2}\left\{ tr\left( \left( AA^T + \Sigma \right)^{-1} C \right) + \log\left( \det\left( AA^T + \Sigma \right) \right) + m \log 2\pi \right\} \quad \cdots \quad (22)$$

Here, n is the number of pieces of data x, m is the number of independent components, an operator tr is a trace (sum of diagonal elements) of a matrix, and an operator det is a determinant. In addition, C is a sample covariance matrix obtained by sample calculation from the data x, and is calculated by the following Expression.

$$C = \frac{1}{n}\sum_{i=1}^{n} x_i x_i^T \quad \cdots \quad (23)$$

The mixing matrix A and the covariance matrix $\Sigma$ of noise can be calculated from the maximum likelihood method using the logarithmic likelihood function L (A, E) of the above Expression (22). As the mixing matrix A, it is possible to obtain a matrix that is hardly influenced by the random noise $\rho$ of the above Expression (20). This is the basic principle of the FA. In addition, as the algorithm of the FA, there are various algorithms using the algorithm other than the maximum likelihood method. Also in the present embodiment, it is possible to use such various kinds of FA.

Incidentally, the estimate obtained by the FA is just the value of $AA^T$. When the mixing matrix A suitable for this value is determined, it is possible to de-correlate the data while reducing the influence of random noise. However, since the degree of freedom of rotation remains, it is not possible to determine each of the plurality of components $s_i$ uniquely. On the other hand, the ICA is processing for reducing the degree of freedom of rotation of the plurality of components $s_i$ so that the plurality of components $s_i$ are perpendicular to each other. In the present embodiment, therefore, the value of the mixing matrix A calculated by the FA is used as a whitening matrix (matrix after whitening), and the arbitrary property with respect to the left rotation is specified by the ICA. Thus, by performing the ICA after performing the whitening processing robust against noise, it is possible to determine the independent component $s_i$ perpendicular to each other. In addition, as a result of such processing, it is possible to improve the measurement accuracy regarding the component $s_i$ by reducing the influence of random noise.

The FA can be considered to be an extension corresponding to the noise of the PCA. In the FA, as a precondition for this extension, it is assumed that noise is normally distributed. This assumption is reasonable in many cases, and better performance can be expected. However, depending on the characteristics of the observation data, the accuracy may not be stable or may not be improved by the FA from the reason that the noise distribution deviates from the normal distribution, for example. In this case, it is appropriate to perform the known process using the PCA.

C-3. ICA (kurtosis as an independence indicator)

Generally, in the independent component analysis (ICA), a high-order statistic indicating the independence of separated pieces of data is used as an indicator for the separation of independent components (independence indicator) . Kurtosis is a typical independence indicator. The ICA using kurtosis as an independence indicator is described in detail in chapter 8 of Aapo Hyvarinen, Juha Karhumen, Erkki Oja, "Independent Comonent Analysis", 2001, John Wiley & Sons, Inc., for example.

Evaluation of the influence on the measurement accuracy according to the selection of pre-processing

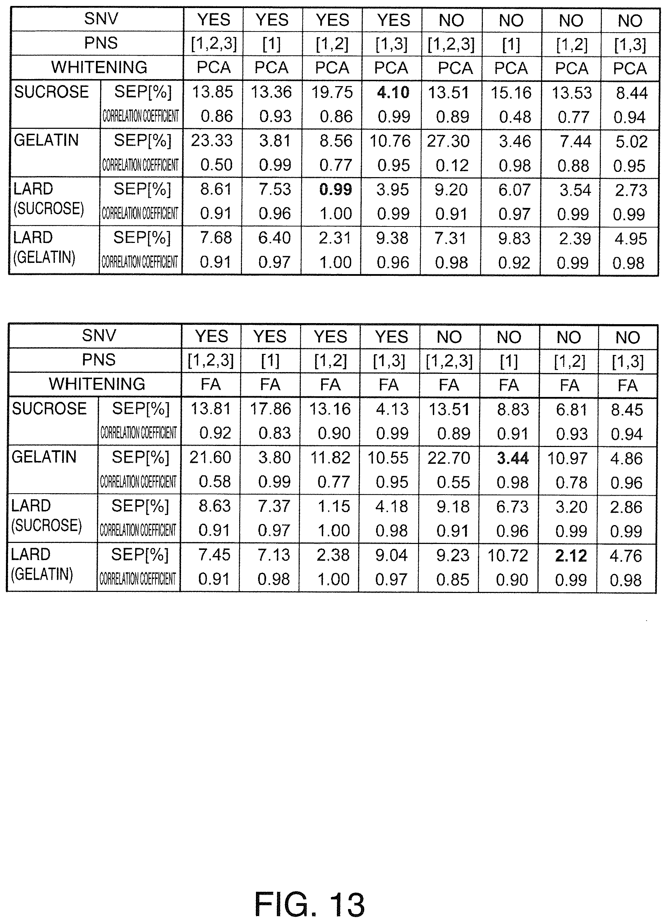

Fig. 13 summarizes the results of accuracy evaluation when measuring one substance from a sample, in which three substances of sucrose, gelatin, and lard are mixed, for each selectable pre-processing.

D. Modification examples

The invention is not limited to the above-described embodiment or modification examples thereof, but various modifications may be made within the scope without departing from the subject matter or the spirit of the invention. For example, the following modification examples are also possible.

Modification example 1

In the embodiment described above, the subject observation data acquisition unit 510 (Fig. 11) acquires the independent component matrix Y including an independent component corresponding to the target component by acquiring the data set for measurement DS2 from the hard disk drive 30, and the mixing coefficient calculation unit 530 (Fig. 11) calculates the estimated mixing matrix AA for the subj ect based on the independent component matrix Y and the absorbance spectrum of the subject and calculates the mixing coefficient of the target component for the subj ect by extracting the mixing coefficient $\alpha_k$ of the k-th column corresponding to the target component rank k from the estimated mixing matrix AA. However, the invention is not limited to this. For example, it is possible to adopt the following configuration in which (i) and (ii) are performed in order.

(i) The data set for measurement DS2 stored in the hard disk drive 30 is read, and an element (independent component) $Y_k$ of the k-th column corresponding to the target component rank k is acquired from the independent component matrix Y included in the data set for measurement DS2. The independent component $Y_k$ has the highest correlation to the chlorophyll content, and corresponds to the chlorophyll content.
(ii) Subsequently, an inner product of the extracted independent component $Y_k$ and the spectrum Xp (for example, the normalized spectrum obtained in step S320) of the subject that is observation data is calculated, and the inner product value is set as the mixing coefficient $\alpha_k$ of the target component. That is, calculation according to the following Expression (24) is performed.

$$\alpha_k \; = \; X_p \cdot Y_k \; \cdots \; (24)$$

Here, the observation data is a linear sum of independent components, and it is assumed that the orthogonality of independent components is sufficiently high. Therefore, by calculating the inner product of the independent component matrix of the target component and the spectrum that is observation data, only the values of the independent components remain and all of the other components become 0. As a result, it becomes easy to calculate the mixing coefficient $\alpha_k$ of the target component. However, when the orthogonality of independent components are not sufficiently high, it is preferable to calculate the estimated mixing matrix $\Lambda A$ of Expression (15) without using the calculation of Expression (27) .
In the process (i) described above, the CPU 10 functions as a data-for-measurement acquisition unit. In the process (ii) described above, the CPU 10 functions as a mixing coefficient calculation unit. Instead of the configuration of the above (i), the data-for-measurement acquisition unit may be configured to acquire the independent component $Y_k$ from a storage unit, such as the hard disk drive 30 in which the element (independent component) $Y_k$ of the k-th column corresponding to the target component rank k in the independent component matrix Y is stored in advance. This is because only independent components corresponding to the target component are necessary and other independent components are not necessary when using the inner product. In this case, the independent component becomes a vector, and it is not necessary to store the target component rank.

Modification example 2

In the embodiment and the modification example described above, the chlorophyll content of a subject, which is a green vegetable, is detected. However, instead of the chlorophyll content of the green vegetable, applications to various subjects and target components, such as oleic acid in meat and collagen in the skin. In short, if a sample having the same components as a subject is prepared to create a calibration curve, it is possible to correspond to various subjects and target components. In the embodiment and each modification example described above, a configuration is adopted in which measurement is performed with the absorbance spectrum as observation data. However, even if sound data in which sound emitted from a plurality of sound sources is mixed is used as the observation data instead of the absorbance spectrum, it is possible to measure the magnitude of the sound from the specific sound source with the same configuration. In short, in the case of a signal having a sufficient amount of information to know the statistical properties of the signal source, the invention can be applied to various kinds of observation data.

Modification example 3

In the embodiment and each modification example described above, in the mixing coefficient estimation step, an independent component matrix is calculated, an estimated mixing matrix is calculated, and a mixing coefficient corresponding to the target component is extracted from the estimated mixing matrix. However, this configuration does not necessarily need to be adopted. In short, it is possible to adopt any configuration in which each independent component, which is included in observation data of each sample, when dividing the observation data into a plurality of independent components is estimated and a mixing coefficient corresponding to the target component is calculated for each sample based on each independent component.

Modification example 4

In the calibration curve creation methods of the embodiment and each modification example described above, the content of the target component in each sample is measured. However, instead of this configuration, it is also possible to prepare a sample containing a target component whose content is known and input the content through a keyboard or the like.

Modification example 5

In the embodiment and each modification example described above, the number of elements m of the spectrum S of an unknown component is determined experimentally or empirically in advance. However, the number of elements m of the spectrum S of the unknown component may also be determined according to the information criteria known as Minimum Description Length (MDL) or Akaike Information Criteria (AIC) . When the MDL or the like is used, the number of elements m of the spectrum S of the unknown component can be automatically determined by calculation from the observation data of the sample. In addition, the MDL is described in "Independent component analysis for noisy data - MEG data analysis, 2000", for example.

Modification example 6

In the embodiment and each modification example described above, a subject that is the target of the measurement process has the same components as a sample used when creating the calibration curve. However, when calculating the mixing coefficient using an inner product as in the modification example 1, an unknown component other than the same component as the sample used when creating the calibration curve may be contained in the subject. Since the inner product of independent components is assumed to be 0, the inner product of independent components corresponding to the unknown component can also be considered to be 0. Therefore, the influence of the unknown component can be neglected when calculating the mixing coefficient using an inner product.

Modification example 7

The computer used in the embodiment and each modification can be replaced with a dedicated apparatus instead of a personal computer. For example, the personal computer to realize the target component measuring method can be replaced with a dedicated gauging apparatus.

Modification example 8

In the embodiment described above, the input of the spectrum of the spectral reflectance of a sample or a subject is performed by inputting the spectrum measured by the spectrometer. However, the invention is not limited to this. For example, it is also possible to estimate a spectrum from a plurality of band images having different wavelength bands and input this spectrum. The band images are obtained by imaging a sample or a subject using a multi-band camera including a filter capable of changing the transmission wavelength band.

Modification example 9

In the embodiment and each modification example described above, the function realized by software may also be realized by hardware.

In addition, elements in the embodiment and each modification example described above, which are not elements mentioned in the appended independent claims, are additional elements, and may be appropriately omitted.

Modification example 10

In the embodiment described above, as a pre-processing selection method, a method of selecting the optimal pre-processing by repeating the selection of pre-processing in step 4 and the evaluation in step 7 is adopted. However, it is possible to use other methods. For example, the operator may select pre-processing in step 4, and step 7 may not be performed.

**Claims**

**1.** A calibration curve creation method of creating a calibration curve, which is used to derive a content of a target component in a subject, from observation data of the subject, comprising:

(a) acquiring the observation data for a plurality of samples of the subject;
(b) acquiring the content of the target component in each sample;
(c) executing pre-processing for the observation data of each sample, a pre-processing method is selected from a plurality of options;
(d) estimating a plurality of independent components when separating the pre-processed observation data of each sample into a plurality of independent components and calculating a mixing coefficient corresponding to the target component for each sample based on the plurality of independent components;
(e) calculating a regression equation of the calibration curve based on the content of the target component of each of the plurality of samples and the mixing coefficient of each sample; and
(f) evaluating accuracy of the calibration curve;

wherein, in the process (c), the pre-processing includes first pre-processing including processing for correcting the observation data and second pre-processing including whitening, and a plurality of processing methods are prepared as processing methods of each of the first pre-processing and the second pre-processing and the pre-processing method is set by combining one or more of the processing methods of each of the first pre-processing and the second pre-processing,
the process (d) includes:

(i) calculating an independent component matrix including the independent component of each sample;
(ii) calculating an estimated mixing matrix, which indicates a set of vectors defining a ratio of an independent component element of each independent component in each sample, from the independent component matrix ; and
(iii) calculating a correlation between each of the vectors included in the estimated mixing matrix and the content of the target component of each of the plurality of samples and selecting the vector, which is determined to have the highest correlation, as a mixing coefficient corresponding to the target component,

in the process (i), the first pre-processing, the second pre-processing, and independent component analysis processing are excuted in this order using the pre-processing method selected in the process (c), and
processes (c) to (f) are repeated to select a plurality of combinations of pre-processing, evaluate the accuracy for each of said combinations, and select an optimal combination among said combinations.

**2.** The calibration curve creation method according to claim 1,
wherein, in the process (c), the processing methods of the first pre-processing include a projection on null space .

**3.** The calibration curve creation method according to claim 1 or 2,
wherein, in the process (c), the processing methods of the first pre-processing include centering.

**4.** The calibration curve creation method according to any one of the preceding claims,

wherein, in the process (c), the processing methods of the first pre-processing include normalization.

5. The calibration curve creation method according to any one of the preceding claims,
wherein, in the process (c), the processing methods of the first pre-processing include smoothing processing.

6. The calibration curve creation method according to any one of the preceding claims,
wherein, in the process (c), the processing methods of the first pre-processing include differential spectrum processing.

7. The calibration curve creation method according to any one of the preceding claims,
wherein, in the process (c), the processing methods of the first pre-processing include differential processing.

8. The calibration curve creation method according to any one of the preceding claims,
wherein, in the process (c), the processing methods of the second pre-processing include a principal component analysis.

9. The calibration curve creation method according to any one of the preceding claims,
wherein, in the process (c), the processing methods of the second pre-processing include a factor analysis.

10. A calibration curve creation apparatus (400) configured to create a calibration curve, which is used to derive a content of a target component in a subject, from observation data of the subject, comprising:

a sample observation data acquisition unit (410) configured to acquire the observation data for a plurality of samples of the subject;
a sample target component amount acquisition unit (420) configured to acquire the content of the target component in each sample;
a pre-processing method selection unit (430) configured to select a processing method of a pre-processing of the observation data from a plurality of options, wherein the pre-processing includes first pre-processing including correction processing and second pre-processing including whitening;
a mixing coefficient estimation unit (440) configured to estimate a plurality of independent components when separating the observation data of each sample into a plurality of independent components and calculate a mixing coefficient corresponding to the target component for each sample based on the plurality of independent components;
a regression equation calculation unit (450) configured to calculate a regression equation of the calibration curve based on the content of the target component of each of the plurality of samples and the mixing coefficient of each sample; and
an algorithm evaluation unit (460) configured to evaluate accuracy of the calibration curve,
wherein, among a plurality of processing methods prepared as processing methods of each of the first pre-processing and the second pre-processing, the pre-processing method selection unit (430) is configured to combine one or more of the processing methods of each of the first pre-processing and the second pre-processing to set the pre-processing method having a plurality of options and select an optimal combination from the set pre-processing method,
the mixing coefficient estimation unit (440) includes:

an independent component matrix calculation section (442) configured to calculate an independent component matrix including the independent component of each sample;
an estimated mixing matrix calculation section (444) configured to calculate an estimated mixing matrix, which indicates a set of vectors defining a ratio of an independent component element of each independent component in each sample, from the independent component matrix; and
a mixing coefficient selection section (446) configured to calculate a correlation between each of the vectors included in the estimated mixing matrix and the content of the target component of each of the plurality of samples and selects the vector, which is determined to have the highest correlation, as a mixing coefficient corresponding to the target component,

the independent component matrix calculation section (442) is configured to calculate the independent component matrix by executing the first pre-processing, the second pre-processing, and independent component analysis processing in this order using the pre-processing method selected by the pre--processing method selection unit (430), and

the pre-processing method selection unit (430), the mixing coefficient estimation unit (440), the regression equation calculation unit (450), and the algorithm evaluation unit (460) are configured to function repeatedly to select a plurality of combinations of pre-processing, evaluate the accuracy for each of said combinations, and select the optimal combination among said combinations.

11. The calibration curve creation apparatus (400) according to claim 10, further comprising:

a storage unit (20) configured to store the independent component matrix calculated by the independent component matrix calculation section (442), a target component rank indicating at which position of the estimated mixing matrix the mixing coefficient selected by the mixing coefficient selection section (446) is present, and a regression equation calculated by the regression equation calculation unit (450) .

**Patentansprüche**

1. Kalibrierkurvenerzeugungsverfahren eines Erzeugens einer Kalibrierkurve, die dafür verwendet wird, einen Inhalt einer Zielkomponente in einem Subjekt abzuleiten, aus Beobachtungsdaten des Subjekts, umfassend:

(a) Erfassen der Beobachtungsdaten für eine Vielzahl von Proben des Subjekts;
(b) Erfassen des Inhalts der Zielkomponente in jeder Probe;
(c) Ausführen einer Vorverarbeitung für die Beobachtungsdaten jeder Probe, wobei ein Vorverarbeitungsverfahren aus einer Vielzahl von Optionen ausgewählt wird;
(d) Abschätzen einer Vielzahl unabhängiger Komponenten beim Separieren der vorverarbeiteten Beobachtungsdaten jeder Probe in eine Vielzahl von unabhängigen Komponenten und Berechnen eines mit der Zielkomponente korrespondierenden Mischkoeffizienten für jede Probe basierend auf der Vielzahl von unabhängigen Komponenten;
(e) Berechnen einer Regressionsgleichung der Kalibrierkurve basierend auf dem Inhalt der Zielkomponente jeder aus der Vielzahl von Proben und dem Mischkoeffizienten jeder Probe; und
(f) Auswerten von Genauigkeit der Kalibrierkurve;

wobei in dem Prozess (c) die Vorverarbeitung erstes Vorverarbeiten, das ein Verarbeiten zum Korrigieren der Beobachtungsdaten aufweist, und zweites Vorverarbeiten, das Weißen aufweist, aufweist, und eine Vielzahl von Verarbeitungsverfahren als Verarbeitungsverfahren jedes des ersten Vorverarbeitens und des zweiten Vorverarbeitens vorbereitet werden und das Vorverarbeitungsverfahren durch Kombinieren einer oder mehrerer der Verarbeitungsverfahren jedes des ersten Vorverarbeitens und des zweiten Vorverarbeitens eingestellt wird, der Prozess (d) aufweist:

(i) Berechnen einer Unabhängige-Komponente-Matrix, die die unabhängige Komponente jeder Probe aufweist;
(ii) Berechnen einer abgeschätzten Mischmatrix, die einen Satz von Vektoren anzeigt, die ein Verhältnis eines unabhängigen Komponentenelements jeder unabhängigen Komponente in jeder Probe definieren, aus der Unabhängige-Komponente-Matrix; und
(iii) Berechnen einer Korrelation zwischen jedem der in der abgeschätzten Mischmatrix enthaltenen Vektoren und dem Inhalt der Zielkomponente von jeder aus der Vielzahl von Proben und Auswählen des Vektors, der ermittelt wird, die höchste Korrelation zu haben, als einen Mischkoeffizienten, der mit der Zielkomponente korrespondiert,

in dem Prozess (i) das erste Vorverarbeiten, das zweite Vorverarbeiten und unabhängige Komponentenanalyseverarbeitung in dieser Reihenfolge unter Verwendung des in dem Prozess (c) ausgewählten Vorverarbeitungsverfahrens ausgeführt werden, und
Prozesse (c) bis (f) wiederholt werden, um eine Vielzahl von Kombinationen von Vorverarbeitung auszuwählen, die Genauigkeit für jede der Kombinationen auszuwerten, und eine optimale Kombination unter den Kombinationen auszuwählen.

2. Kalibrierkurvenerzeugungsverfahren nach Anspruch 1,
wobei im Prozess (c) die Verarbeitungsverfahren des ersten Vorverarbeitens eine Projektion auf Nullraum aufweisen.

3. Kalibrierkurvenerzeugungsverfahren nach Anspruch 1 oder 2,
wobei im Prozess (c) die Verarbeitungsverfahren des ersten Vorverarbeitens Zentrieren aufweisen.

**4.** Kalibrierkurvenerzeugungsverfahren nach einem der vorstehenden Ansprüche, wobei im Prozess (c) die Verarbeitungsverfahren des ersten Vorverarbeitens Normalisierung aufweisen.

**5.** Kalibrierkurvenerzeugungsverfahren nach einem der vorstehenden Ansprüche, wobei im Prozess (c) die Verarbeitungsverfahren des ersten Vorverarbeitens Glättungsverarbeitung aufweisen.

**6.** Kalibrierkurvenerzeugungsverfahren nach einem der vorstehenden Ansprüche, wobei im Prozess (c) die Verarbeitungsverfahren des ersten Vorverarbeitens Differenzspektrenverarbeitung aufweisen.

**7.** Kalibrierkurvenerzeugungsverfahren nach einem der vorstehenden Ansprüche, wobei im Prozess (c) die Verarbeitungsverfahren des ersten Vorverarbeitens Differenzverarbeitung aufweisen.

**8.** Kalibrierkurvenerzeugungsverfahren nach einem der vorstehenden Ansprüche, wobei im Prozess (c) die Verarbeitungsverfahren des zweiten Vorverarbeitens eine Hauptkomponentenanalyse aufweisen.

**9.** Kalibrierkurvenerzeugungsverfahren nach einem der vorstehenden Ansprüche, wobei im Prozess (c) die Verarbeitungsverfahren des zweiten Vorverarbeitens eine Faktoranalyse aufweisen.

**10.** Kalibrierkurvenerzeugungsvorrichtung (400), die dafür eingerichtet ist, eine Kalibrierkurve, die dafür verwendet wird, einen Inhalt einer Zielkomponente in einem Subjekt abzuleiten, aus Beobachtungsdaten des Subjekts zu erzeugen, umfassend:

eine Probenbeobachtungsdatenerfassungseinheit (410), die dafür eingerichtet ist, die Beobachtungsdaten für eine Vielzahl von Proben des Subjekts zu erfassen;
eine Probenzielkomponentenmengenerfassungseinheit (420), die dafür eingerichtet ist, den Inhalt der Zielkomponente in jeder Probe zu erfassen;
eine Vorverarbeitungsverfahrenauswahleinheit (430), die dafür eingerichtet ist, ein Vorverarbeitungsverfahren einer Vorverarbeitung der Beobachtungsdaten aus einer Vielzahl von Optionen auszuwählen, wobei die Vorverarbeitung erstes Vorverarbeiten, das Korrekturverarbeiten aufweist, und zweitens Vorverarbeiten, das Weißen aufweist, aufweist;
eine Mischkoeffizientabschätzungseinheit (440), die dafür eingerichtet ist, eine Vielzahl von unabhängigen Komponenten beim Separieren der vorverarbeiteten Beobachtungsdaten jeder Probe in eine Vielzahl von unabhängigen Komponenten abzuschätzen und einen mit der Zielkomponente korrespondierenden Mischkoeffizienten für jede Probe basierend auf der Vielzahl von unabhängigen Komponenten zu berechnen;
eine Regressionsgleichungsberechnungseinheit (450), die dafür eingerichtet ist, eine Regressionsgleichung der Kalibrierkurve basierend auf dem Inhalt der Zielkomponente jeder aus der Vielzahl von Proben und dem Mischkoeffizienten jeder Probe zu berechnen; und
eine Algorithmusauswertungseinheit (460), die dafür eingerichtet ist, die Genauigkeit der Kalibrierkurve auszuwerten;
wobei, unter einer Vielzahl von Verarbeitungsverfahren, die als Verarbeitungsverfahren jedes des ersten Vorverarbeitens und des zweiten Vorverarbeitens vorbereitet sind, die Vorverarbeitungsverfahrenauswahleinheit (430) dafür eingerichtet ist, eine oder mehrere der Verarbeitungsverfahren jedes des ersten Vorverarbeitens und des zweiten Vorverarbeitens zu kombinieren, um das Vorverarbeitungsverfahren, das eine Vielzahl von Optionen hat, einzustellen und eine optimale Kombination von dem eingestellten Vorverarbeitungsverfahren auszuwählen,
wobei die Mischkoeffizientabschätzungseinheit (440) aufweist:

einen Unabhängige-Komponente-Matrix-Berechnungsabschnitt (442), der dafür eingerichtet ist, eine Unabhängige-Komponente-Matrix, die die unabhängige Komponente jeder Probe aufweist, zu berechnen;
einen Abgeschätzte-Mischmatrix-Berechnungsabschnitt (444), der dafür eingerichtet ist, aus der Unabhängige-Komponente-Matrix eine abgeschätzte Mischmatrix zu berechnen, die einen Satz von Vektoren anzeigt, die ein Verhältnis eines unabhängigen Komponentenelements jeder unabhängigen Komponente in jeder Probe definiert; und
einen Mischkoeffizientauswahlabschnitt (446), der dafür eingerichtet ist, eine Korrelation zwischen jedem der in der abgeschätzten Mischmatrix enthaltenen Vektoren und dem Inhalt der Zielkomponente von jeder aus der Vielzahl von Proben zu berechnen, und den Vektor, der ermittelt wird, die höchste Korrelation zu haben, als einen Mischkoeffizienten, der mit der Zielkomponente korrespondiert, auswählt,
wobei der Unabhängige-Komponente-Matrix-Berechnungsabschnitt (442) dafür eingerichtet ist, die Unabhängige-Komponente-Matrix durch Ausführen des ersten Vorverarbeitens, des zweiten Vorverarbeitens und unabhängiger Komponentenanalyseverarbeitung in dieser Reihenfolge unter Verwendung des durch die Vorverarbeitungsverfahrenauswahleinheit (430) ausgewählten Vorverarbeitungsverfahrens zu berechnen, und
die Vorverarbeitungsverfahrenauswahleinheit (430), die Mischkoeffizientabschätzungseinheit (440), die Regressionsgleichungsberechnungseinheit (450) und die Algorithmusauswertungseinheit (460) dafür eingerichtet sind, wiederholt zu arbeiten, um eine Vielzahl von Kombinationen von Vorverarbeitung auszuwählen, die Genauigkeit für jede der Kombinationen auszuwerten, und die optimale Kombination unter den Kombinationen auszuwählen.

11. Kalibrierkurvenerzeugungsvorrichtung (400) nach Anspruch 10, weiter umfassend:

eine Speichereinheit (20), die dafür eingerichtet ist, die durch den Unabhängige-Komponente-Matrix-Berechnungsabschnitt (442) berechnete Unabhängige-Komponente-Matrix, einen Zielkomponenterang, der anzeigt, an welcher Position der abgeschätzten Mischmatrix der durch den Mischkoeffizientauswahlabschnitt (446) ausgewählte Mischkoeffizient vorhanden ist, und eine Regressionsgleichung, die durch die Regressionsgleichungsberechnungseinheit (450) berechnet wird, zu speichern.

**Revendications**

1. Procédé de création de courbe d'étalonnage consistant à créer une courbe d'étalonnage, qui est utilisée pour dériver une teneur d'un composant cible chez un sujet, à partir de données d'observation du sujet, comprenant :

(a) l'acquisition des données d'observation pour une pluralité d'échantillons du sujet ;
(b) l'acquisition de la teneur du composant cible dans chaque échantillon ;
(c) l'exécution d'un prétraitement pour les données d'observation de chaque échantillon, un procédé de prétraitement est sélectionné parmi une pluralité d'options ;
(d) l'estimation d'une pluralité de composants indépendants lors de la séparation des données d'observation prétraitées de chaque échantillon en une pluralité de composants indépendants et le calcul d'un coefficient de mélange correspondant au composant cible pour chaque échantillon sur la base de la pluralité de composants indépendants ;
(e) le calcul d'une équation de régression de la courbe d'étalonnage sur la base de la teneur du composant cible de chacun de la pluralité d'échantillons et du coefficient de mélange de chaque échantillon ; et
(f) l'évaluation de la précision de la courbe d'étalonnage ;

dans lequel, dans le processus (c), le prétraitement inclut un premier prétraitement incluant un traitement pour corriger les données d'observation et un second prétraitement incluant un blanchiment, et une pluralité de procédés de traitement sont préparés en tant que procédés de traitement de chacun du premier prétraitement et du second prétraitement et le procédé de prétraitement est établi par la combinaison d'un ou de plusieurs procédés de traitement de chacun du premier prétraitement et du second prétraitement,
le processus (d) inclut :

(i) le calcul d'une matrice de composants indépendants incluant le composant indépendant de chaque échantillon ;
(ii) le calcul d'une matrice de mélange estimé, qui indique un ensemble de vecteurs définissant un taux d'un élément de composant indépendant de chaque composant indépendant dans chaque échantillon, à partir de la matrice de composants indépendants ; et
(iii) le calcul d'une corrélation entre chacun des vecteurs inclus dans la matrice de mélange estimé et la teneur du composant cible de chacun de la pluralité d'échantillons et la sélection du vecteur, qui est déterminé pour avoir la corrélation la plus élevée, en tant que coefficient de mélange correspondant au composant cible,

dans le processus (i), le premier prétraitement, le second prétraitement, et un traitement d'analyse de composant indépendant sont exécutés dans cet ordre en utilisant le procédé de prétraitement sélectionné dans le processus (c), et
les processus (c) à (f) sont répétés pour sélectionner une pluralité de combinaisons de prétraitements, évaluer la précision pour chacune desdites combinaisons, et sélectionner une combinaison optimale parmi lesdites combinaisons.

2. Procédé de création de courbe d'étalonnage selon la revendication 1,
dans lequel, dans le processus (c), les procédés de traitement du premier prétraitement incluent une projection sur un espace nul.

3. Procédé de création de courbe d'étalonnage selon la revendication 1 ou 2,
dans lequel, dans le processus (c), les procédés de traitement du premier prétraitement incluent un centrage.

4. Procédé de création de courbe d'étalonnage selon l'une quelconque des revendications précédentes,
dans lequel, dans le processus (c), les procédés de traitement du premier prétraitement incluent une normalisation.

5. Procédé de création de courbe d'étalonnage selon l'une quelconque des revendications précédentes,
dans lequel, dans le processus (c), les procédés de traitement du premier prétraitement incluent un traitement de lissage.

6. Procédé de création de courbe d'étalonnage selon l'une quelconque des revendications précédentes,
dans lequel, dans le processus (c), les procédés de traitement du premier prétraitement incluent un traitement de spectre différentiel.

7. Procédé de création de courbe d'étalonnage selon l'une quelconque des revendications précédentes,
dans lequel, dans le processus (c), les procédés de traitement du premier prétraitement incluent un traitement différentiel.

8. Procédé de création de courbe d'étalonnage selon l'une quelconque des revendications précédentes,
dans lequel, dans le processus (c), les procédés de traitement du second prétraitement incluent une analyse de composant principal.

9. Procédé de création de courbe d'étalonnage selon l'une quelconque des revendications précédentes,
dans lequel, dans le processus (c), les procédés de traitement du second prétraitement incluent une analyse de facteur.

10. Appareil de création de courbe d'étalonnage (400) conçu pour créer une courbe d'étalonnage, qui est utilisée pour dériver une teneur d'un composant cible chez un sujet, à partir de données d'observation du sujet, comprenant :

une unité d'acquisition de données d'observation d'échantillon (410) conçue pour acquérir les données d'observation pour une pluralité d'échantillons du sujet;
une unité d'acquisition de quantité de composant cible d'échantillon (420) conçue pour acquérir la teneur du composant cible dans chaque échantillon ;
une unité de sélection de procédé de prétraitement (430) conçue pour sélectionner un procédé de traitement d'un prétraitement des données d'observation parmi une pluralité d'options, dans lequel le prétraitement inclut un premier prétraitement incluant un traitement de correction et un second prétraitement incluant un blanchissement;
une unité d'estimation de coefficient de mélange (440) conçue pour estimer une pluralité de composants indépendants lors de la séparation des données d'observation de chaque échantillon en une pluralité de composants indépendants et calculer un coefficient de mélange correspondant au composant cible pour chaque échantillon sur la base de la pluralité de composants indépendants;
une unité de calcul d'équation de régression (450) conçue pour calculer une équation de régression de la courbe d'étalonnage sur la base de la teneur du composant cible de chacun de la pluralité d'échantillons et du coefficient de mélange de chaque échantillon ; et
une unité d'évaluation d'algorithme (460) conçue pour évaluer la précision de la courbe d'étalonnage,
dans lequel, parmi une pluralité de procédés de traitement préparés en tant que procédés de traitement de chacun du premier prétraitement et du second prétraitement, l'unité de sélection de procédé de prétraitement (430) est conçue pour combiner un ou plusieurs des procédés de traitement de chacun du premier prétraitement et du second prétraitement afin d'établir le procédé de prétraitement présentant une pluralité d'options et de sélectionner une combinaison optimale parmi le procédé de prétraitement établi,
l'unité d'estimation de coefficient de mélange (440) inclut :

une section de calcul de matrice de composants indépendants (442) conçue pour calculer une matrice de composants indépendants inclut le composant indépendant de chaque échantillon ;

une section de calcul de matrice de mélange estimé (444) conçue pour calculer une matrice de mélange estimé, qui indique un ensemble de vecteurs définissant un taux d'un élément de composant indépendant de chaque composant indépendant dans chaque échantillon, à partir de la matrice de composants indépendants; et

une section de sélection de coefficient de mélange (446) conçue pour calculer une corrélation entre chacun des vecteurs inclus dans la matrice de mélange estimé et la teneur du composant cible de chacun de la pluralité d'échantillons et sélectionner le vecteur, qui est déterminé pour avoir la corrélation la plus élevée, en tant que coefficient de mélange correspondant au composant cible,

la section de calcul de matrice de composants indépendants (442) est conçue pour calculer la matrice de composants indépendants par l'exécution du premier prétraitement, du second prétraitement, et d'un traitement d'analyse de composant indépendant dans cet ordre en utilisant le procédé de prétraitement sélectionné par l'unité de sélection de procédé de prétraitement (430), et

l'unité de sélection de procédé de prétraitement (430), l'unité d'estimation de coefficient de mélange (440), l'unité de calcul d'équation de régression (450), et l'unité d'évaluation d'algorithme (460) sont conçues pour fonctionner à plusieurs reprises afin de sélectionner une pluralité de combinaisons de prétraitements, d'évaluer la précision pour chacune desdites combinaisons, et de sélectionner la combinaison optimale parmi lesdites combinaisons.

11. Appareil de création de courbe d'étalonnage (400) selon la revendication 10, comprenant en outre :

une unité de stockage (20) conçue pour stocker la matrice de composants indépendants calculée par la section de calcul de matrice de composants indépendants (442), un classement de composants cibles indiquant la position dans la matrice de mélange estimé où le coefficient de mélange sélectionné par la section de sélection de coefficient de mélange (446) est présent, et une équation de régression calculée par l'unité de calcul d'équation de régression (450).

EP 2 784 484 B1

START

STEP 1 — PREPARATION

STEP 2 — MEASUREMENT OF SPECTRUM

STEP 3 — MEASUREMENT OF CHLOROPHYLL CONTENT

STEP 4 — SELECTION OF PRE-PROCESSING

STEP 5 — ESTIMATION OF MIXING COEFFICIENT

STEP 6 — CALCULATION OF REGRESSION EQUATION

STEP 7 — EVALUATION OF ALGORITHM

IS THERE ANY PRE-PROCESSING THAT HAS NOT BEEN EVALUATED ? — YES

NO

END

# FIG. 1

29

FIG. 2

FIG. 3A

400

410

SAMPLE OBSERVATION DATA
ACQUISITION UNIT

420

SAMPLE TARGET COMPONENT
AMOUNT ACQUISITION UNIT

430

PRE-PROCESSING
SELECTION UNIT

440

MIXING COEFFICIENT
ESTIMATION UNIT

442

INDEPENDENT COMPONENT
MATRIX CALCULATION SECTION

444

ESTIMATED MIXING MATRIX
CALCULATION SECTION

446

MIXING COEFFICIENT
SELECTION SECTION

450

REGRESSION EQUATION
CALCULATION UNIT

460

ALGORITHM EVALUATION UNIT

# FIG. 3B

442

┌─────────────────────────────────────────────────────────────────┐
│ INDEPENDENT COMPONENT                                             │
│ MATRIX CALCULATION SECTION                                        │
│                                                        470         │
│  ┌───────────────────────────────────────────────────────────┐   │
│  │          FIRST PRE-PROCESSING SECTION                      │   │
│  │        (NORMALIZATION PROCESSING SECTION)                  │   │
│  │                                                            │   │
│  │      472                              474                  │   │
│  │  ┌────────────────────────┐   ┌────────────────────────┐  │   │
│  │  │   STANDARD NORMAL      │   │   PROJECTION ON         │  │   │
│  │  │ VARIATE TRANSFORMATION │   │   NULL SPACE            │  │   │
│  │  │        (SNV)           │   │     (PNS)               │  │   │
│  │  └────────────────────────┘   └────────────────────────┘  │   │
│  └───────────────────────────────────────────────────────────┘   │
│                              │                                    │
│                              ▼          480                        │
│  ┌───────────────────────────────────────────────────────────┐   │
│  │          SECOND PRE-PROCESSING SECTION                     │   │
│  │         (WHITENING PROCESSING SECTION)                     │   │
│  │                                                            │   │
│  │      482                              484                  │   │
│  │  ┌────────────────────────┐   ┌────────────────────────┐  │   │
│  │  │  PRINCIPAL COMPONENT   │   │                         │  │   │
│  │  │      ANALYSIS          │   │   FACTOR ANALYSIS       │  │   │
│  │  │        (PCA)           │   │        (FA)             │  │   │
│  │  └────────────────────────┘   └────────────────────────┘  │   │
│  └───────────────────────────────────────────────────────────┘   │
│                              │                                    │
│                              ▼          490                        │
│  ┌───────────────────────────────────────────────────────────┐   │
│  │          INDEPENDENT COMPONENT ANALYSIS                    │   │
│  │                PROCESSING SECTION                          │   │
│  │            (ICA PROCESSING SECTION)                        │   │
│  │                                                            │   │
│  └───────────────────────────────────────────────────────────┘   │
│                                                                   │
└─────────────────────────────────────────────────────────────────┘

# FIG. 3C

|  | FIRST PRE-PROCESSING | SECOND PRE-PROCESSING |
|---|---|---|
| 1 | PNS [1,2,3]+SNV | PCA |
| 2 | PNS [1,2]+SNV | PCA |
| 3 | PNS [1,3]+SNV | PCA |
| 4 | PNS [1]+SNV | PCA |
| 5 | PNS [1,2,3] | PCA |
| 6 | PNS [1,2] | PCA |
| 7 | PNS [1,3] | PCA |
| 8 | PNS [1] | PCA |
| 9 | PNS [1,2,3]+SNV | FA |
| 10 | PNS [1,2]+SNV | FA |
| 11 | PNS [1,3]+SNV | FA |
| 12 | PNS [1]+SNV | FA |
| 13 | PNS [1,2,3] | FA |
| 14 | PNS [1,2] | FA |
| 15 | PNS [1,3] | FA |
| 16 | PNS [1] | FA |

# FIG. 4

DS1

| SAMPLE NUMBER | CHLOROPHYLL CONTENT | SPECTRAL DATA |
|---|---|---|
| B1 | C1 | SPECTRUM $X_1$ <br> ABSORBANCE <br> WAVELENGTH |
| B2 | C2 | SPECTRUM $X_2$ <br> ABSORBANCE <br> WAVELENGTH |
| ⋮ | ⋮ | ⋮ |
| Bn | Cn | SPECTRUM $X_n$ <br> ABSORBANCE <br> WAVELENGTH |

# FIG. 5

```
        ┌─────────────────────────────┐
        │   MIXING COEFFICIENT        │
        │   ESTIMATION PROCESS        │
        └─────────────────────────────┘
                      │
                      ▼
┌─────────────────────────────────────────────┐
│  INDEPENDENT COMPONENT ANALYSIS BASED         │
│         ON SPECTRAL DATA X                     │──── S110
│  (CALCULATION OF SEPARATION MATRIX W)          │
└─────────────────────────────────────────────┘
                      │
                      ▼
┌─────────────────────────────────────────────┐
│  CALCULATE INDEPENDENT COMPONENT MATRIX Y      │
│  ON THE BASIS OF SEPARATION MATRIX W AND       │──── S120
│            SPECTRAL DATA X                      │
│             (Y = W · X)                         │
└─────────────────────────────────────────────┘
                      │
                      ▼
┌─────────────────────────────────────────────┐
│  CALCULATE ESTIMATED MIXING MATRIX Â           │
│  ON THE BASIS OF SPECTRAL DATA X AND           │──── S130
│  INDEPENDENT COMPONENT MATRIX Y                │
│             (Â = X · Y⁺)                        │
└─────────────────────────────────────────────┘
                      │
                      ▼
┌─────────────────────────────────────────────┐
│  CALCULATE CORRELATION COEFFICIENT R           │
│  BETWEEN CHLOROPHYLL CONTENT C AND             │
│  COMPONENT (VECTOR α̂) OF EACH COLUMN           │──── S140
│  INCLUDED IN ESTIMATED MIXING MATRIX Â         │
└─────────────────────────────────────────────┘
                      │
                      ▼
┌─────────────────────────────────────────────┐
│  EXTRACT VECTOR α̂ RELEVANT TO                  │
│  HIGHEST CORRELATION COEFFICIENT R             │──── S150
└─────────────────────────────────────────────┘
                      │
                      ▼
              ┌──────────────┐
              │    RETURN     │
              └──────────────┘
```

# FIG. 6

INDEPENDENT
COMPONENT

TB

SAMPLE
NUMBER

| | $Y_1$ | $Y_2$ | $\cdots$ | $Y_m$ |
|---|---|---|---|---|
| $B_1$ | $\hat{a}_{11}$ | $\hat{a}_{12}$ | $\cdots$ | $\hat{a}_{1m}$ |
| $B_2$ | $\hat{a}_{21}$ | $\hat{a}_{22}$ | $\cdots$ | $\hat{a}_{2m}$ |
| $\vdots$ | $\vdots$ | $\vdots$ | $\cdots$ | $\vdots$ |
| $B_n$ | $\hat{a}_{n1}$ | $\hat{a}_{n2}$ | $\cdots$ | $\hat{a}_{nm}$ |

TARGET COMPONENT
RANK k = 2

# FIG. 7

FIG. 8

FIG. 9

```
┌─────────────────────────────┐
│   REGRESSION EQUATION       │
│   CALCULATION PROCESS       │
└─────────────────────────────┘
               │
               ▼
┌───────────────────────────────────────────────────────┐
│ CALCULATES REGRESSION EQUATION ON THE BASIS OF         │──S210
│ CHLOROPHYLL CONTENT C AND EXTRACTED VECTOR α̂           │
└───────────────────────────────────────────────────────┘
               │
               ▼
┌───────────────────────────────────────────────────────┐
│ STORE CONSTANTS u AND v OF REGRESSION EQUATION,        │
│ TARGET COMPONENT RANK k, AND INDEPENDENT               │──S220
│ COMPONENT MATRIX Y                                     │
└───────────────────────────────────────────────────────┘
               │
               ▼
          ┌──────────┐
          │  RETURN  │
          └──────────┘
```

# FIG. 10

```
                                                    ┌500
┌──────────────────────────────────────────────────────┐
│                                                        │
│                                          ┌510          │
│        ┌──────────────────────────────────┐           │
│        │ SUBJECT OBSERVATION DATA          │           │
│        │ ACQUISITION UNIT                  │           │
│        └──────────────────────────────────┘           │
│                                                        │
│                                          ┌520          │
│        ┌──────────────────────────────────┐           │
│        │ DATA-FOR-MEASUREMENT              │           │
│        │ ACQUISITION UNIT                  │           │
│        └──────────────────────────────────┘           │
│                                                        │
│                                          ┌530          │
│        ┌──────────────────────────────────┐           │
│        │ MIXING COEFFICIENT                │           │
│        │ CALCULATION UNIT                  │           │
│        └──────────────────────────────────┘           │
│                                                        │
│                                          ┌540          │
│        ┌──────────────────────────────────┐           │
│        │ TARGET COMPONENT AMOUNT           │           │
│        │ CALCULATION UNIT                  │           │
│        └──────────────────────────────────┘           │
│                                                        │
└──────────────────────────────────────────────────────┘
```

# FIG. 11

```
        ╭─────────────────────────╮
        │   TARGET COMPONENT      │
        │  MEASURING PROCESS      │
        ╰─────────────────────────╯
                    │
                    ▼
┌─────────────────────────────────────────┐
│                                         │
│       MEASURE SPECTRUM OF SUBJECT       │──── S310
│                                         │
└─────────────────────────────────────────┘
                    │
                    ▼
┌─────────────────────────────────────────┐
│                                         │
│    ACQUIRE DATA SET FOR MEASUREMENT DS2  │──── S315
│                                         │
└─────────────────────────────────────────┘
                    │
                    ▼
┌─────────────────────────────────────────┐
│                                         │
│       PRE-PROCESSING ON SPECTRUM        │──── S325
│                                         │
└─────────────────────────────────────────┘
                    │
                    ▼
┌─────────────────────────────────────────┐
│    CALCULATE ESTIMATED MIXING MATRIX Â  │
│  ON THE BASIS OF PRE-PROCESSED SPECTRUM AND │──── S335
│ INDEPENDENT COMPONENT MATRIX Y OF DATA SET │
│          FOR MEASUREMENT DS2            │
└─────────────────────────────────────────┘
                    │
                    ▼
┌─────────────────────────────────────────┐
│   SELECT ESTIMATED MIXING COEFFICIENT α̂k │
│   OF k-TH COLUMN CORRESPONDING TO TARGET │──── S340
│ COMPONENT RANK k FROM ESTIMATED MIXING MATRIX Â │
└─────────────────────────────────────────┘
                    │
                    ▼
┌─────────────────────────────────────────┐
│ CALCULATE CONTENT OF CHLOROPHYLL ON THE BASIS OF │
│  CONSTANTS u AND v OF REGRESSION EQUATION AND │──── S350
│    MIXING COEFFICIENT α̂k OF CHLOROPHYLL  │
└─────────────────────────────────────────┘
                    │
                    ▼
            ╭───────────────╮
            │    RETURN      │
            ╰───────────────╯
```

# FIG. 12

| SNV | | YES | YES | YES | YES | NO | NO | NO | NO |
|---|---|---|---|---|---|---|---|---|---|
| PNS | | [1,2,3] | [1] | [1,2] | [1,3] | [1,2,3] | [1] | [1,2] | [1,3] |
| WHITENING | | PCA | PCA | PCA | PCA | PCA | PCA | PCA | PCA |
| SUCROSE | SEP[%] | 13.85 | 13.36 | 19.75 | **4.10** | 13.51 | 15.16 | 13.53 | 8.44 |
| | CORRELATION COEFFICIENT | 0.86 | 0.93 | 0.86 | 0.99 | 0.89 | 0.48 | 0.77 | 0.94 |
| GELATIN | SEP[%] | 23.33 | 3.81 | 8.56 | 10.76 | 27.30 | 3.46 | 7.44 | 5.02 |
| | CORRELATION COEFFICIENT | 0.50 | 0.99 | 0.77 | 0.95 | 0.12 | 0.98 | 0.88 | 0.95 |
| LARD (SUCROSE) | SEP[%] | 8.61 | 7.53 | **0.99** | 3.95 | 9.20 | 6.07 | 3.54 | 2.73 |
| | CORRELATION COEFFICIENT | 0.91 | 0.96 | 1.00 | 0.99 | 0.91 | 0.97 | 0.99 | 0.99 |
| LARD (GELATIN) | SEP[%] | 7.68 | 6.40 | 2.31 | 9.38 | 7.31 | 9.83 | 2.39 | 4.95 |
| | CORRELATION COEFFICIENT | 0.91 | 0.97 | 1.00 | 0.96 | 0.98 | 0.92 | 0.99 | 0.98 |

| SNV | | YES | YES | YES | YES | NO | NO | NO | NO |
|---|---|---|---|---|---|---|---|---|---|
| PNS | | [1,2,3] | [1] | [1,2] | [1,3] | [1,2,3] | [1] | [1,2] | [1,3] |
| WHITENING | | FA | FA | FA | FA | FA | FA | FA | FA |
| SUCROSE | SEP[%] | 13.81 | 17.86 | 13.16 | 4.13 | 13.51 | 8.83 | 6.81 | 8.45 |
| | CORRELATION COEFFICIENT | 0.92 | 0.83 | 0.90 | 0.99 | 0.89 | 0.91 | 0.93 | 0.94 |
| GELATIN | SEP[%] | 21.60 | 3.80 | 11.82 | 10.55 | 22.70 | **3.44** | 10.97 | 4.86 |
| | CORRELATION COEFFICIENT | 0.58 | 0.99 | 0.77 | 0.95 | 0.55 | 0.98 | 0.78 | 0.96 |
| LARD (SUCROSE) | SEP[%] | 8.63 | 7.37 | 1.15 | 4.18 | 9.18 | 6.73 | 3.20 | 2.86 |
| | CORRELATION COEFFICIENT | 0.91 | 0.97 | 1.00 | 0.98 | 0.91 | 0.96 | 0.99 | 0.99 |
| LARD (GELATIN) | SEP[%] | 7.45 | 7.13 | 2.38 | 9.04 | 9.23 | 10.72 | **2.12** | 4.76 |
| | CORRELATION COEFFICIENT | 0.91 | 0.98 | 1.00 | 0.97 | 0.85 | 0.90 | 0.99 | 0.98 |

# FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007044104 A

- JP 2001099710 A

### Non-patent literature cited in the description

- **TOIVIAINEN, M.** Blind source separation in diffuse reflectance NIR spectroscopy using independent component analysis. *J. Chemometrics,* 2010, vol. 24, 514-522
- **ROGER, M. JARVIS.** Genetic algorithm optimization for pre-processing and variable selection of spectroscopic data. *Bioinformatics,* 2005, vol. 21 (7), 860-868

- **ZENG-PING CHEN ; JULIAN MORRIS ; ELAINE MARTIN.** *Extracting Chemical Information from Spectral Data with Multiplicative Light Scattering Effects by Optical Path-Length Estimation and Correction,* 2006
- **AAPO HYVARINEN ; JUHA KARHUMEN ; ERKKI OJA.** Independent Comonent Analysis. John Wiley & Sons, Inc, 2001